# EUROPEAN PATENT APPLICATION

(11) **EP 2 591 823 A1**
(43) Date of publication of application: **15.05.2013**
(21) Application number: 11188841.8
(22) Date of filing: 11.11.2011
(51) Int. Cl.: A61P 25/28, A61K 36/23, A61K 36/59

(54) **Composition for treating or preventing neurodegenerative disorders**

(71) Applicant: Biologische Heilmittel Heel GmbH, 76532 Baden-Baden (DE)
(72) Inventor: Seilheimer, Bernd., 76530 Baden-Baden (DE); Röska, Kerstin., 97244 Bütthard (DE)
(74) Representative: Dick, Alexander

(57) **Abstract**

The present invention is concerned with plant derived pharmaceutically useful compositions. In particular, it relates to a composition for use in treating and/or preventing a neurodegenerative disorder or disease, wherein said composition comprises extracts of Conium maculatum, Ambra grisea, Petroleum rectificatum, and Anamirta cocculus extracts from the and wherein the composition provides the said extracts in a dosage of at least about 1 ml/kg body weight of the subject to be treated per day. The present invention further relates to said composition for use in improving learning and memory. Contemplated is also a medicament comprising said composition as well as a method for treating and/or preventing a neurodegenerative disorder or disease in a subject suffering therefrom, said method comprises administering in a therapeutically effective amount to the said subject a composition.

## Description

The present invention is concerned with plant derived pharmaceutically useful compositions. In particular, it relates to a composition for use in treating and/or preventing a neurodegenerative disorder or disease, wherein said composition comprises Conium maculatum, Ambra grisea, Petroleum rectificatum, and Anamirta cocculus extracts from the and wherein the composition provides the said extracts in a dosage of at least about 1 ml/kg body weight of the subject to be treated per day. The present invention further relates to said composition for use in improving learning and memory. Contemplated is also a medicament comprising said composition as well as a method for treating and/or preventing a neurodegenerative disorder or disease in a subject suffering therefrom, said method comprises administering in a therapeutically effective amount to the said subject a composition.

Neurodegenerative diseases and disorders are currently in focus of various scientific and medical investigations. Due to unknown etiology it is very difficult to cure such complex and multifactorial CNS diseases.. They are emerging diseases occurring more frequently in an elderly population. As a consequence of the demografic development of our society to a more older population, there is a high medical need to cure such diseases. Accordingly, there are various efforts made today to prevent or treat such disease more efficiently. However to date the therapeutic options are limited to symptomatic treatment. Only drugs which reduces symptoms but no diseases-modifying drugs are authorized for neurodegenerative diseases.

A neurodegenerative disease of particular importance in the developed countries with an elderly becoming society is Alzheimer's disease. Alzheimer's disease is at present the most common causeof dementia. It is an incurable, progressive, degenerative, and terminal disease that was first described by Alois Alzheimer in early 20th century. It is the fourth common cause of death following cardiovascular disease cancer and stroke. Alzheimer's diesease is in most cases diagnosed at an age of more than 65 years. However, the onset of the diesease may occur much earlier. From an epidemiological view the disease is crucial, too, since about 26 million patients suffered worldwide from Alzheimer's disease in 2006 and there is a prognosis that 1 in 85 people globally will suffer from said diesease by 2050. The etiology of Alzheimers disease is still unknown but great progress has been made to understand the cellular and molecular mechanisms of this multifactorial disease. It is suggested that Alzheimer's disease is triggered by multiple events leading to a convergent and complex pathophysiology (Bolognesi 2009, Curr Pharm Des 15(6): 601-613, Leon 2011, Curr Med Chem 18(4): 552-576. Galimberti 2011, CNS Neurol Disord Drug Targets 10(2): 163-174, Niedermeyer 2011, Am J Electroneurodiagnostic Technol 51(2): 82-91).

The course of Alzheimer's disease is individually different for all patients. However, there are some common symptoms, the earliest of which are most often misinterpreted as being caused by other influences such as age or stress conditions. Alzheimer's disease is characterized by three hallmarks: Diffuse neuronal loss mainly in the cholinergic system, intracellular protein deposits called neurofibrillary tangles and extracellular amyloid plaques deposition. These factors have been suggested as the cause of Alzheimer's disease and they are the basis for different theory explaining the Alzheimer's disease pathogenesis such as the cholinergic hypothesis (Bartus 1982, Science. 1982 Jul 30;217(4558):408-14), the amyloide cascade hypothesis (Hardy 1992, Trends Neurosci. 1992 Jun;15(6):200-1) and the hyperphosphorylation hypothesis (Grundke-Iqbal 1986, J Alzheimers Dis. 2006;9(3 Suppl):219-42). In its early stages, a common symptom of the disease is a reduced memory, in particular with respect to more recent events (short term memory). The disease can be confirmed by MRT or PET scanning of the brain and behavioral analyses as well as cognitive tests.

Advanced symptoms of the disease include confusion, irritability, aggression, mood swings, language breakdown, long-term memory loss, and the general withdrawal of the patient as the senses become more and more impaired. In a final stage, important body functions become impaired due to the neuronal degeneration and this impairment finally leads to death.

Alzheimer's disease has a poor prognosis and most patients do not survive more than ten years after the first diagnosis was established. Disease management is a great challenge since a special type of care is required for the patients.

The causes and factors influencing the disease are not yet well understood. The disease was originally identified by histopathological findings, i.e. the presence of so called plaque structures in the brain. There is strong scientific evidence that the processing of the amyloid beta precursor protein (APP) is impaired in Alzheimer patients and this impairment produces molecular species such as amyloid beta protein (Aß) or oligomeres thereof such as amyloid-derived diffusible ligands (ADDL) which affect the neuronal communication (Palop 2010, Neuromolecular Med. 2010 Mar; 12(1):48-55., Zhang 2011, APP processing in Alzheimer's disease. Mol Brain 4: 3, Duyckaerts 2009, Acta Neuropathol. 2009 Jul;118(1):5-36).

Currently significant efforts are undertaken to develop drugs against Alzheimer's disease as well as other neurodegenerative disorders. For example, in 2008, there have been about 500 clinical trials for Alzheimer drugs. Unfortunately, due to toxicity concerns and inefficacy of drugs the development of new therapeutic entities is limited, only symptomatic treatments and no disease modifying medications are currently available.

Besides the aforementioned Alzheimer's disease, there are further severe neurodegenerative diseases which also involve an impaired neuronal communication. Such impaired neuronal communication must not necessarily result in cognitive impairment but may also affect other body functions governed by the nervous system. Examples for the aforementioned diseases include amyotrophic lateral sclerosis (Sica 2011, Arq Neuropsiquiatr. 2011 Aug;69(4):699-706) circumscribed brain atrophy, corticobasale degeneration, degeneration of nervous system due to alcohol, unspecified degenerative disease of nervous system, degenerative syndrome, diabetic polyneuropathy, epilepsy, Friedreich's ataxias, frontotoemporale dementia and Parkinsonism of Chromosom 17, Huntington's disease (Vonsattel 2011, Handb Clin Neurol. 2011;100:83-100), mild cognitive impairment, Morbus Pick, non-classified degenerative diseases or disorders of nervous system, progressive isolated aphasia, progressive supranuclear palsy, non-classified senile degeneration of brain, spinocerebellar ataxias/degenerations, mental retardation, spinal cord injury, forgetfullness, and Parkinson's disease (Coskun 2011, Biochim Biophys Acta. 2011 Aug 16, Gruber 2011, Postepy Hig Med Dosw (Online). 2011 Aug 19;65:542-51).

Phytopharmaceuticals have been reported as being effective medications in many disease areas already. Phytopharmaceuticals are, e.g., known for a long time as homeopathic drugs. Homeopathic drugs are highly diluted preparations of naturally occurring ingredients, most often plant derived compounds or complex mixtures thereof. Phytopharmaceuticals from Ginko biloba have been mentioned as potential drugs for the treatment of dementia. However, the pharmaceutical efficacy is still put into jeopardy. Similarly, phytodrugs such as Resveratrol, Ginseng, and Green Tea Extract have been reported as drugs for the treatment of neurodegenrative disorders.

More complex phytopharmaceuticals such as Vertigoheel® have been reported to improve the microcirculation in general and, thereby, could be beneficial for improving some symptoms accompanying neurodegenerative disease at homeopathic dosage (see, e.g., Issing 2005, J Alter Compl Med 11(1): 155-160; Klopp 2005, Microvascular Rev 69: 10-16; Weiser 1998, Arch Otolaryngol Head Nech Surg 124: 879-885; Schneider 2005, Arzneim.-Forsch/Drug Res 55(1): 23-29); Heinle 2010, Clinical hemorheology and microcirculation 46, 23-25; Wolscher 2001, Biologische Medizin, 30, 184-190; Seeger-Schellerhof 2009, J Integrative Medicine 1:231; Zenner s. Biological Ther. X:281-288, 1992.

However, there is still a need for further compositions for efficiently treating and/or preventing neurodegenerative diseases or disorders.

The technical problem underlying the present invention can be seen as the provision of means and methods for complying with the aforementioned needs. The technical problem is solved by the embodiments characterized in the claims and herein below.

Accordingly, the present invention relates to a composition for use in treating and/or preventing a neurodegenerative disorder or disease, wherein said composition comprises extracts of Conium maculatum obtainable according to method 2a of the German Homeopathic Pharmacopeia, Ambra grisea obtainable according to the information in the Ambra grisea monograph of the German Homeopathic Pharmacopeia, Petroleum rectificatum obtainable according to method 5a and additional information in the Petroleum rectificatum monograph of the German Homeopathic Pharmacopeia, and Anamirta cocculus obtainable according to method 4a and additional information in the Anamirta cocculus monograph of the German Homeopathic Pharmacopeia and wherein the composition provides the said extracts in a concentration of at least about 1 ml/kg body weight of the subject to be treated per day.

The term "composition" as used herein refers to a mixture of extracts from, inter alia, biological sources such as plants which are further defined elsewhere herein. Preferably, the said composition can further comprise other ingredients or diluents. Preferably, such further ingredients can be stabilizing agents, wetting agents, pharmaceutical carriers, additional pharmaceutically active agents, release controlling agents and the like. Preferred diluents encompass water, alcohols, physiological saline solutions, buffers, such as phosphate buffered saline solutions, syrup, oil, water, emulsions, various types of wetting agents, and the like. Preferably envisaged in accordance with the present invention is a composition which further comprises at least one pharmaceutically acceptable carrier and/or diluent. The carrier(s) must be acceptable in the sense of being compatible with the other ingredients of the formulation and being not deleterious to the recipient thereof. The pharmaceutical carrier employed may include a solid, a semi-solid (e.g., a gel), or a liquid. Exemplary of solid carriers are lactose, terra alba, sucrose, talc, gelatin, agar, pectin, acacia, magnesium stearate, stearic acid and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The pharmaceutically acceptable diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers and the like.

The composition shall be adapted for use in treating and/or preventing a neurodegenerative disease or disorder. Accordingly, it will be understood that dependent on the desired mode of administration the composition shall be formulated for a systemic or topical application. Preferably, the composition envisaged herein is formulated for a systemic oral application. However, depending on the nature and the mode of action, the composition may be administered by other routes as well including intra-muscular, intraperitoneally, subcutaneous, oral, or intravenous administration. The composition can be, preferably, formulated for a bolus administration or can be made for continuous applications as set forth elsewhere herein in detail. Preferably, the composition according to the present invention is formulated as a medicament as set forth elsewhere herein in detail.

The term "treating" as used herein refers to any improvement of the neurodegenerative disease or disorder that occurs in a treated subj ect compared to an untreated subj ect. Such an improvement can be a prevention of a worsening or progression of the said disease or disorder. Moreover, such an improvement may also be an amelioration or cure of the neurodegenerative disease or disorder or its accompanying symptoms. It will be understood that a treatment may not be successful for 100% of the subjects to be treated. The term, however, requires that the treatment is successful for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test, one-way-ANOVA following post hoc tests, two-way-ANOVA, Wilcoxon Test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.05, 0.01, 0.005, or 0.0001

The term "preventing" as used herein refers to avoiding the onset of the neurodegenerative disease or disorder as used herein or its accompanying syndromes. It will be understood that prevention refers to avoiding the onset of a neurodegenerative disease or disorder within a certain time window in the future. Said time window shall, preferably, start upon administration of a compound in the sense of the invention and lasts for at least 1 month, at least 6 months, at least 9 months, at least 1 year, at least 2 years, at least 5 years, at least 10 years or even for the remaining physiological life span of a subject. It will be understood that a prevention may not be successful for 100% of the subjects to be treated for the prevention. The term, however, requires that the prevention is successful for a statistically significant portion of the subjects (e.g. a cohort in a cohort study). Whether a portion is statistically significant can be determined without further ado by the person skilled in the art using various well known statistic evaluation tools discussed also elsewhere herein in detail.

The term "neurodegenerative disease or disorder" as used herein refers to any disease or disorder which is caused by an impaired neuronal outgrowth, by neuronal death due to apoptosis, intoxication or necrosis, or by impaired neuronal function due to dendritic or axonal defects. Preferably, said neurodegenerative disorder or disease is associated with cognitive impairment. More preferably, said cognitive impairment is prevented or ameliorated by the composition of the present invention. Preferably, the neuronal outgrowth is increased upon administration of the composition.

Accordingly, preferred neurodegenerative diseases or disorders referred to in accordance with the present invention. are Alzheimer's disease, amnesia, learning and memory deficits, amyotrophic lateral sclerosis, circumscribed brain atrophy, corticobasale degeneration, degeneration of nervous system due to alcohol, unspecified degenerative disease of nervous system, degenerative syndrome, diabetic polyneuropathy, epilepsy, Friedreich's ataxias, frontotoemporale dementia and Parkinsonism of Chromosom 17, Huntington's disease, mild cognitive impairment, Morbus Pick, non-classified degenerative diseases or disorders of nervous system, progressive isolated aphasia, progressive supranuclear palsy, non-classified senile degeneration of brain, spinocerebellar ataxias/degenerations, mental retardation, spinal cord injury, forgetfullness, and Parkinson's disease. The symptoms accompanying the aforementioned diseases and disorders are well known to those skilled in the art and are described in more detail in standard text books of medicine such as Stedman or Pschyrembl.

In a particular preferred embodiment of the composition of the invention, said composition is for use in treating and/or preventing Alzheimer's disease as neurodegenerative disease or disorder. Alzheimer's disease is known to be accompanied by a an improper processing of the amyloid-beta precursor protein (APP). Preferably, amyloid-beta precursor protein (APP) processing accompanying Alzheimer's disease is reduced upon administration of the composition of the present invention.

The plants referred to in accordance with the present invention, i.e. Conium maculatum and Anamirta cocculus, are well known to the person skilled in the art and botanically characterized. Accordingly, the said plants as a starting material for preparing the composition according to the present invention can be identified, cultured and/or harvested without further ado. In particular, the plants are, preferably, obtained by growing developing seedlings in the green house under standard conditions of humidity, temperature, and illumination into plants, culturing said plants for a period of time sufficient to allow for production of secondary plant metabolites and harvesting the plants or parts thereof required for the extraction process defined elsewhere herein..

Conium maculatum to be used for the preparation of the composition according to the invention is an alcoholic extract obtainable according to method 2a of the German Homeopathic Pharmacopeia. How to carry out the method 2a according to the German Homeopathic Pharmacopeia is well known in the art. Preferably, the Conium macculatum extract to be used in accordance with the composition of the present invention is prepared as follows: A mother tincture is obtained from fresh Conium maculatum herbs containing less than 70% of expressed juice and more than 60% moisture (loss during drying) and no essential oil or resin. Suitable raw herbal material is subsequently comminuted at 105°C for 2 hours in a desiccator. The comminuted herbal material is admixed with an amount of ethanol (90% vol/vol) not less than half of the mass of the herbal material. The precise amount of ethanol to be added is to be calculated according to the following formula: mass of raw herbal material (kg) multiplied by the percentage loss on drying (this can be determined by analyzing the loss during drying for a sample of the herbal material) divided by 100. The mixture is subsequently stored in closed containers at room temperature (not exceeding 20°C) for at least 10 days with swirling from time to time. The mixture is than expressed and the resulting liquid is filtered. The filtrate is adjusted with ethanol (50% vol/vol). The amount of ethanol for said adjustment is calculated as follows: mass of filtrate (kg) multiplied by [percentage dry residue or percentage assay content of the filtrate minus percentage of dry residue or assay content required according to the monograph] divided by percentage of dry residue or assay content required according to the monograph. The adjusted mixture is allowed to stand at room temperature (not exceeding 20°C) for not less than 5 days and, optionally, filtered again to yield the mother tincture. D-dilutions of the mother tincture can be made by applying the following dilution scheme: 2 parts of mother tincture are admixed with 8 parts of ethanol (50% vol/vol) to obtain a D1 dilution. 1 part of the D1 dilution is admixed with 9 parts of ethanol (50% vol/vol) in order to obtain a D2 dilution.

Preferably, the Conium macculatum extract to be used for the composition according to the invention is characterized by the analytical parameters set forth in the respective monograph. The Conium macculatum extract is, preferably, provided in form of a mother tincture or a D2 dilution. The aforementioned Conium macculatum extract is comprised in the composition according to the invention, preferably, in a D3 dilution and in a portion of 10 % (w/w).

Anamirta cocculus to be used for the preparation of the composition according to the invention is an alcoholic extract obtainable according to obtainable according to method 4a and additional information in the Anamirta cocculus monograph of the German Homeopathic Pharmacopeia. How to carry out the method according to the German Homeopathic Pharmacopeia is well known in the art. Preferably, the Anamirta cocculus extract to be used in accordance with the composition of the present invention is prepared as follows: The herbal raw material is comminuted and 10 parts of ethanol (90% vol/vol) is per 1 part of dried herbal material. The mixture is subsequently stored in closed containers at room temperature (not exceeding 20°C) for sufficient time. The residues are separated from the ethanol and pressed out. The two liquids thereby obtained are combined as macerate. For percolation, a mixture is prepared as described above and stored in closed containers at room temperature (not exceeding 20°C) for sufficient time. The mixture is transferred to a percolator and the percolate is allowed to flow slowly at room temperature such that the herbal material is always covered with the remaining ethanol. Residues may be pressed out and the expressed liquid is combined with the percolate. The percolate and the macerate are combined and adjusted with ethanol (90% vol/vol). The amount of ethanol of the appropriate concentration for adjustment is calculated by the following formula: mass of percolate or macerate (kg) multiplied by [percentage dry residue or percentage assay content of the percolate or macerate minus percentage of dry residue or assay content required according to the monograph] divided by percentage of dry residue or assay content required according to the monograph. The resulting adjusted mixture of the percolate and macerate is the mother tincture corresponding also to the D1 dilution. A D2 dilution is obtainable by admixing 1 part of the mother tincture/D 1 dilution with 9 parts of ethanol of the appropriate concentration.

Preferably, the Anamirta cocculus extract to be used for the composition according to the invention is characterized by the analytical parameters set forth in the respective monograph. The Anamirta cocculus extract is, preferably, provided in form of a mother tincture or a D3 dilution. The aforementioned Anamirta cocculus extract is comprised in the composition according to the invention, preferably, in a D4 dilution in a portion of 70 % (w/w).

Petroleum rectificatum obtainable according to method 5a and additional information in the Petroleum rectificatum monograph of the German Homeopathic Pharmacopeia is a purified fraction of petroleum obtained from petroleum crude oil by a sequential distillation process called rectification. How to obtain petroleum rectificatum is well known in the art and described in the aforementioned Petroleum rectificatum monograph. Serial D-dilutions can be prepared by as follows: A D1 dilution is obtained by admixing 1 part of Petroleum rectificatum with 9 parts of ethanol 90% (vol/vol). One part of the D1 dilution is subsequently admixed with 9 parts of ethanol (50% vol/vol) in order to obtain a D2 dilution. The subsequent dilutions, e.g., the D3 to D7 dilutions are obtainable as described for the D2 dilution using in each case the previous dilution as a starting material.

Preferably, petroleum rectificatum to be used for the composition according to the present invention is characterized by the analytical parameters set forth in the respective monograph. Petroleum rectificatum is, preferably, used in a D8 dilution. Petroleum rectifictum is comprised in the composition according to the invention in a portion of about 10 % (w/w).

Ambra grisea obtainable according to the information in the Ambra grisea monograph of the German Homeopathic Pharmacopeia is a substance present in the gut of pot whale. How to obtain Ambra grisea is well known in the art and described in the aforementioned Ambra grisea monograph. In particular, 10 parts of crushed Ambra material are admixed with 100 parts of water-free ethanol and the mixture is heated up to the boiling point for 1 hour in a reflux condenser. After the mixture is cooled down, it will be filtered in order to obtain the mother tincture which also corresponds to the D1 dilution. The serial dilutions D2 to D4 are obtainable by admixing 1 part of the mother tincture or previous dilution with 9 parts of ethanol (90% vol/vol). From dilution D5 onwards, 1 part of the previous dilution is admixed with 9 parts of ethanol (50% vol/vol).

Preferably, Ambra grisea to be used for the composition according to the present invention is characterized by the analytical parameters set forth in the respective monograph: Ambra grisea is, preferably, used in a D6 dilution. Ambra grisea is comprised in the composition according to the invention, preferably, in a portion of about 10 % (w/w).

Preferably, the composition to be applied according to the present invention is prepared by admixing the aforementioned ingredients as follows: For a 100 g liquid composition, 10 g of Ambra grisea in D6 dilution is admixed with 70 g of Anamirta cocculus in a D4 dilution, 10 g of Conium maculatum in a D3 dilution, and 10 g of Petroleum rectificatum in a D8 dilution. Such a composition can be used for treating and/or preventing a neurodegenerative disease according the present invention. More preferably, said composition Vertigoheel® applied in ultra high dosages, i.e. a dosage which is at least about 60-fold to 130 fold, preferably at least 64 fold or at least 127-fold, the recommended dosage of its current homeopathic applications.

The composition shall be formulated and/or used or administered in a manner as to provide the extracts to the subject in a dosage of at least about 1 ml/kg body weight of the subject per day and preferably, a dosage within the range of at least 1 ml/kg body weight per day to about 2 ml/kg body weight per day. Preferably, the aforementioned dosage can be provided by a single administration (bolus) or may be achieved by more than one administration steps within the day. Moreover, it is also envisaged that the dosage is, preferably, provided by using continuous release devices which provide for a continuous administration of the dosage over the day. How the composition can be formulated in a proper manner in order to provide the aforementioned daily dosage is well known in the art. For example, low dosage providing compositions comprising the ingredients of the composition of the present invention and, preferably, the commercially available composition Vertigoheel®, can be administered repeatedly and, thereby, proved the required dosage. Alternatively, a composition being enriched in the individual ingredients can be provided. Such a composition would, preferably, provide the dosage upon a single bolus administration. Preferably, a composition providing the high dose at a single bolus could be provided by increasing the amounts referred to above for the low dose composition for multiple administrations up to at least about 60-fold to 130 fold, preferably at least 64 fold or at least 127-fold.

As discussed before, the composition of the present invention may also encompass further ingredients such as further pharmaceutically active ingredients. Such further ingredients may be comprised in the composition itself, i.e. be a component of the mixture, or may be formulated for a combined application as a physically separate entity such that the actual composition for treating and/or preventing the neurodegenerative disease or disorder is actually formed during the combine administration process in the subject. Preferred further ingredients are compounds or compositions which are currently applied in the therapy or prevention of the aforementioned neurodegenerative diseases or disorders. In particular, the following classes of pharmaceutical ingredients: Acetylcholine esterase inhibitors, NMDA antagonists, angiotensine converting enzyme (ACE) inhibitors, TNF alpha, dopamine precursors, domamine agonists, MAO inhibitors or phytochemicals exhibiting a neuroprotective activity. Preferably, the composition of the present invention comprises at least one further pharmaceutically active ingredient selected from the group consisting of: Donepezil, Rivastigmine, Galantamine, Tacrine, Mementine, Captopril, Etanercept, Gingko Biloba, Vitamine B12, Resveratrol, Ginseng, Green Tea Extract, Vitamine E, Levodopa, Pramipexole dihydrochloride monohydrate, and Rasagiline.

Advantageously, it has been found in the studies underlying the present invention that a composition comprising the aforementioned ingredients can be applied in a certain dosage for the efficient and improved treatment of the neurodegenerative diseases referred to above. In particular, the composition is apparently well suited for the treatment of Alzheimer's disease. Although compositions having the ingredients according to the present invention are known in the art already for low dose applications in homeopathy (e.g., Vertigoheel®), it has been surprisingly found that contrary to the concept of dilutions used in homeopathic approaches, a significant increase in the amount of ingredients administered to a subject results in an efficient treatment and/or prevention of neurodegenerative diseases. In particular, an effective dosage according to the present invention was the approx. 64-fold up to 127 fold dosage used in homeopathic applications, i.e. an ultra high dosage. Further, it was found that in such high doses, the composition effectively and directly stimulates neuronal outgrowth and, thus, restore neuronal function in neurodegenerative disease or disorders. Moreover, the beneficial effects can also be used in apparently healthy subjects for improving learning and memory. Thus, the present invention, further, relates to a composition for use in improving learning and memory, wherein said composition is a composition which comprises extracts of Conium maculatum obtainable according to method 2a of the German Homeopathic Pharmacopeia, Ambra grisea obtainable according to the information in the Ambra grisea monograph of the German Homeopathic Pharmacopeia, Petroleum rectificatum obtainable according to method 5a and additional information in the Petroleum rectificatum monograph of the German Homeopathic Pharmacopeia, and Anamirta cocculus obtainable according to method 4a and additional information in the Anamirta cocculus monograph of the German Homeopathic Pharmacopeia and wherein the composition provides said extracts in a dosage of at least about 1 ml/kg body weight of the subj ect to be treated per day.

The term "learning and memory" as used herein refers to the capability of a subject to analyze and understand a certain context (learning) or to store and remember certain information (memory). Learning as used herein can be improved in that the subject is able to more efficiently analyze or understand a certain context or to understand and analyze a context faster. Memory can be improved by increasing a subjects capacity to store and remember information or by preventing the naturally occurring forgetting. Preferably, natural forgetting is ameliorated in order to improve learning and memory in accordance with the present invention upon administration of the composition. Preferably, the said learning and memory is also improved according to the invention in a subj ect which is apparently healthy at least with respect to neurodegenerative diseases or disorders.

The present invention, furthermore, relates to a medicament comprising a composition which comprises extracts of Conium maculatum obtainable according to method 2a of the German Homeopathic Pharmacopeia, Ambra grisea obtainable according to the information in the Ambra grisea monograph of the German Homeopathic Pharmacopeia, Petroleum rectificatum obtainable according to method 5a and additional information in the Petroleum rectificatum monograph of the German Homeopathic Pharmacopeia, and Anamirta cocculus obtainable according to method 4a and additional information in the Anamirta cocculus monograph of the German Homeopathic Pharmacopeia and wherein the composition provides the said extracts in a dosage of at least about 1 ml/kg body weight of the subject to be treated per day.

The term "medicament" as used herein refers to a pharmaceutical composition containing the aforementioned ingredients in a therapeutically effective dose as referred to elsewhere herein and, preferably. at least one pharmaceutically acceptable carrier and/or diluent. The medicament can be formulated for various routes of administrations set forth elsewhere herein in detail. A therapeutically effective dose refers to amounts of the ingredients to be used for a medicament which prevent, ameliorate or cure the disease or disorders referred to herein or at least symptoms accompanying the said diseases or disorders. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. The specific dosages referred to in this specification are calculated for subject of mid-age and average weight (about 70 kg).

Specific medicaments based on the composition according to the invention are prepared in a manner well known in the pharmaceutical art and comprise the active ingredients referred to herein above in admixture or otherwise associated with at least one pharmaceutically acceptable carrier or diluent. For making those specific medicaments, the active ingredients will usually be mixed and, optionally, combined with a carrier or the diluent. The resulting formulations are to be adapted to the mode of administration. The procedures for formulating a medicament as referred to herein may involve mixing, granulating, compression, or dissolving the ingredients as appropriate to form the desired composition. It will be appreciated that the form and character of the pharmaceutical acceptable carrier or diluent is dictated by the amount of active ingredient with which it is to be combined, the route of administration, and other well-known variables. Dosage recommendations shall be indicated in the prescribers or users instructions in order to anticipate dose adjustments depending on the considered recipient. Finally, it is to be understood that the formulation of a composition according to the invention as a medicament takes place, preferably, under GMP standardized conditions or the like in order to ensure quality, pharmaceutical security, and effectiveness of the medicament.

Thus, the present invention contemplates a method for the manufacture of a medicament, preferably, for use in treating and/or preventing a neurodegenerative disorder or disease comprising the steps of:
(a) preparing a composition which comprises extracts of Conium maculatum obtainable according to method 2a of the German Homeopathic Pharmacopeia, Ambra grisea obtainable according to the information in the Ambra grisea monograph of the German Homeopathic Pharmacopeia, Petroleum rectificatum obtainable according to method 5a and additional information in the Petroleum rectificatum monograph of the German Homeopathic Pharmacopeia, and Anamirta cocculus obtainable according to method 4a and additional information in the Anamirta cocculus monograph of the German Homeopathic Pharmacopeia and wherein the composition provides said extracts in a dosage of at least about 1 ml/kg body weight of the subject to be treated per day; and
(b) formulating said composition as a medicament in a pharmaceutically acceptable manner.

How to prepare the aforementioned extracts of Conium maculatum, Ambra grisea, Petroleum rectificatum, or Anamirta cocculus according to the respective monographs has been set forth elsewhere herein in detail. Moreover, it has been described already how the composition may be formulated as medicament in order to provide the envisaged dosage above.

Finally, the present invention pertains to a method for treating and/or preventing a neurodegenerative disorder or disease in a subject suffering therefrom, said method comprises administering in a therapeutically effective amount to the said subject a composition which comprises extracts of Conium maculatum obtainable according to method 2a of the German Homeopathic Pharmacopeia, Ambra grisea obtainable according to the information in the Ambra grisea monograph of the German Homeopathic Pharmacopeia, Petroleum rectificatum obtainable according to method 5a and additional information in the Petroleum rectificatum monograph of the German Homeopathic Pharmacopeia, and Anamirta cocculus obtainable according to method 4a and additional information in the Anamirta cocculus monograph of the German Homeopathic Pharmacopeia and wherein the composition provides the said extracts in a dosage of at least about 1 ml/kg body weight of the subject to be treated per day.

How to administer the composition referred to above has been described elsewhere herein already in detail. Moreover, the skilled person knows how a therapeutically effective amount can be provided in a suitable form for the present method. Details thereon are found elsewhere in this specification.

All references cited throughout this specification are herewith incorporated by reference with respect to the specific disclosure content referred to above as well as in their entireties.

### FIGURES

Figure 1: Data indicate a dose-and-time-dependent effect of Vertigoheel® on the electrical frequencies in the rat brain via EEG measurement

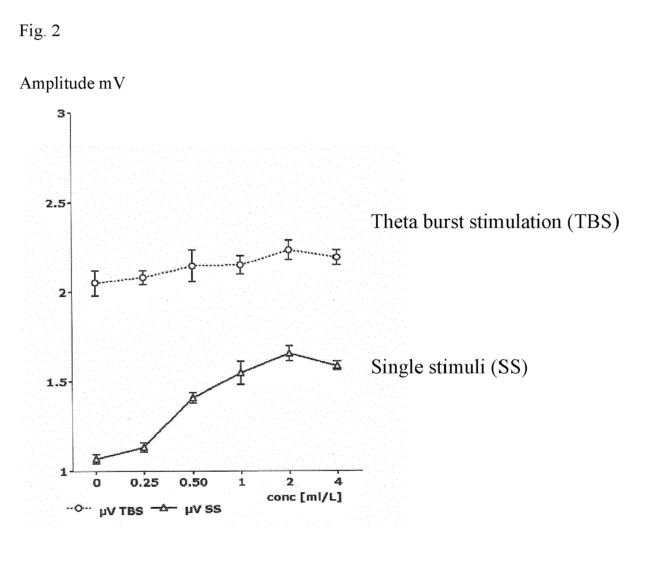

Figure 2: Recording of neuronal activity after single stimulus (SS) and theta burst stimulation (TBS) following Vertigoheel treatment.
Figure 3: Neuronal activity of Vertigoheel® (1ml) in presence of several Glutamate antagonists.
Figure 4: Quantitative analysis of neuronal outgrowth of PHN following Vertigoheel® incubation (1:3 dilution, single treatment). ** P < 0.01, *** P < 0.001 vs. Saline Heel.
Figure 5: sAPP-α Levels following Vertigoheel® treatment in neuroblastoma cell line SHSY-5Y. * P < 0.05, **P < 0.01, *** P < 0.001 vs. Saline Heel.
Figure 6: sAPP-β Levels following Vertigoheel® treatment in neuroblastoma cell line SHSY-5Y. * P < 0.05, **P < 0.01, *** P < 0.001 vs. Saline Heel.
Figure 7: Effect of Vertigoheel® on olfactory memory measured in the Social transmission food preference test, * P < 0.05, *** P < 0.001 in comparison to the corresponding cued food.
Figure 8: Contextual fear conditioning - Effect of Vertigoheel® on memory for cue.
Figure 9: Effect of Vertigoheel® on long term retention memory measured via Passive Avoidance test, * P < 0.05 vs. corresponding control group.
Figure 10: Effect of Vertigoheel® on natural forgetting (time to explore novel object) in the novel object recognition task * P < 0.05 versus 24 h group.
Figure 11: Effect of Vertigoheel® on natural forgetting (number of contacts with novel object) in the novel object recognition task, * P < 0.05 versus 24 h group
Figure 12: Effect of Vertigoheel® on working memory in cognitive deficient mice using T-maze test; *** p < 0.001 versus cognitive deficit group.

### EXAMPLES

### Example 1: Tele EEG Study - Effect of Vertigoheel® on EEG pattern in the rat

It was the objective to characterize Vertigoheel® using the electroencephalographic (EEG)-based electropharmacograms in freely moving rats and to compare the EEG pattern with a series of central nervous system drugs already tested in this system. The determined field potentials contain information about larger local networks of electrically active neurons, reflecting the interaction of drugs with their targets within the concert of neurotransmission. This technique provides insight into the pharmacological time-and-dose-dependent action profile of different preparations, both individually and when compared with each other.

A crossover design with at least a 1-week washout period in between administrations was used in both experimental series. Vertigoheel®, consisting of 4 natural ingredients and physiological saline solution, were used as a control treatment. Three dosages of both preparations were tested: Vertigoheel®, administered by 0.5, 1.0, and 2.0 mL/kg intraperitoneally. The applied dosages are ultra high dosages being at least about 32x, 64x or 127x the dosage applied in the homeopathic applications of the Vertigoheel® composition in humans. After a predose period of 45 minutes for baseline recording, drug effects were observed continuously for 245 minutes, subdivided into 15-minute periods, starting 5 minutes after intraperitoneal administration. Changes of electrical power (µV2) were expressed as percentage of the 45-minute absolute predose spectral power values within each frequency band. Data were averaged from animal recordings for the particular experimental day. Data are expressed as mean values averaged from 10 to 11 animals for each of the 2 successive experimental series.

Twenty-two adult Fisher rats (aged 6-8 months and day-night converted for at least 2 months: 12/12 h beginning at 6 am; purchased from Charles River Laboratories) were implanted with 4 bipolar concentric steel electrodes using a stereotactic surgical procedure. All 4 electrodes were placed 3 mm lateral within the left hemisphere. Dorsoventral coordinates were 4.0, 6.0, 4.2, and 8.0 mm and anterior coordinates were 3.7, 9.7, 5.7, and 12.2 mm for the frontal cortex, striatum, hippocampus, and reticular formation, respectively (according to the atlas of Paxinos). A pre-constructed base plate carrying 4 bipolar stainless steel semimicroelectrodes (neurological electrodes "SNF 100"; Rhodes Medical Instruments, Inc, Summerland, Calif) and a 5-pin plug were fixed to the skull by dental cement interacting with 3 steel screws placed on distance into the bone. The distant recording spot of the electrode was the active electrode, whereas the proximal circular spots of the 4 electrodes were connected to each other to give a common reference. The reticular formation was chosen because it contains the origin of dopaminergic transmission (ventral tegmental area). From there, the nigrostriatal (to striatum), mesolimbic (to hippocampus), and mesocortical (to frontal cortex) pathways were connected. A plug for the transmitter was fixed on the base plate (weight, 5.2 g [including battery]; size, 26×12×6 mm).

During the recording, rats could move freely without receiving food (chewing would have produced too many artefacts). Experiments began at 6:30 am in darkness during the active phase of the animals. Animals were allowed to recover from this procedure for 2 weeks. After this, the transmitter was plugged in for adaptation and control experiments with saline. The principles of laboratory animal care were followed in all trials, and the local authorities responsible for animal care allowed the performance according to German Health Guidelines.

Electroencephalographic signals were recorded from the frontal cortex, hippocampus, striatum, and reticular formation inside a completely copper-shielded room. Signals were wirelessly transmitted by a radiotelemetric system (Rhema Labortechnik, Hofheim, Germany), using 40 MH as the carrier frequency, and were amplified and processed, as previously described, to provide power spectra of 0.25-Hz resolution, according to Dimpfel and Suter. Briefly, after automatic artefact, rejection signals were collected in intervals of 4 seconds and transformed using the fast Fourier technique. The sampling frequency was 512 Hz. Four values were averaged to provide a final sampling frequency of 128 Hz, well above the Nyquist frequency. The resulting electrical power spectra were divided into 6 specially defined frequency ranges: delta, 0.80 to 4.50 Hz; theta, 4.75 to 6.75 Hz; alpha11, 7.00 to 9.50 Hz; alpha2 2, 9.75 to 12.50 Hz; beta1, 12.75 to 18.50 Hz; and beta2, 18.75 to 35.00 Hz. These frequency ranges were recognized to change independently from each other in all earlier trials. Spectra were averaged in 3-minute intervals and displayed on-line. In an off-line procedure, spectra were averaged to obtain 30-minute periods or longer for further analysis and data presentation. With these longer periods, short-term fluctuations of changes were averaged to emphasize pharmacological effects. This type of field potential changes in the presence of drugs compared with baseline and is called an electropharmacogram.

The "Tele-Stereo-EEG" animal model, consisting of continuous recording of intracerebral field potentials, was used in combination with a video tracking system for the detection of changes in motility (GJB Datentechnik GmbH, Langewiesen, Germany). This system recognizes locomotion and stereotyped behaviour by following a contrast difference of the black transmitter on the head of the animal compared with its environment. The system has been validated in a previous study with different dosages of caffeine.

Values were calculated as percentage of the baseline values. These values were log transformed for approaching normal multivariate distribution to fulfil the preconditions of the statistical analysis. The calculation consisted of determining the statistical significance for each frequency band within each brain region, separately, to facilitate interpretation with respect to neurotransmitter receptor interaction. Analysis always compared each period against the identical period of changes after saline administration. Statistics were calculated according to the Wilcoxon (Mann-Whitney) test and discriminant analysis. In this mathematical procedure, drugs with a similar clinical indication group together in space, according to the results of the first 3 discriminant functions, are projected into x-y-z space. First, the 3-dimensional space provides evidence of similarity or dissimilarity of drug action. Second, similar colours suggest a similar mechanism of action. The results of the next 3 discriminant functions are coded in an additive colour mixture of red (fourth function), green (fifth function), and blue (sixth function), which is used to document additional slight differences between the actions of drugs (a well-known method from TV pictures). The position and colour of reference compounds with known clinical indications are listed in the figure, along with dosage and time of recording. Close neighbourhood and similar colour indicate a close similarity of action. According to this system, reference compounds can be grouped via indication-specific activity changes and into clusters with known clinical applications.

### Results

Effects of Saline: After 4 hours of saline administration, following a 45-minute lasting baseline, no significant changes of spectral power within the 4 brain regions were detected.

Effects of Vertigoheel® (ultra high dosage): As a first step, the effect of Vertigoheel® on electrical power was determined. Vertigoheel® exhibited a dose-and-time-dependent effect on the firing rate in the 4 assessed brain areas. During the first hour, the most pronounced effect on spectral frequency changes was measured after treatment with Vertigoheel®, 1 and 2 mL/kg.

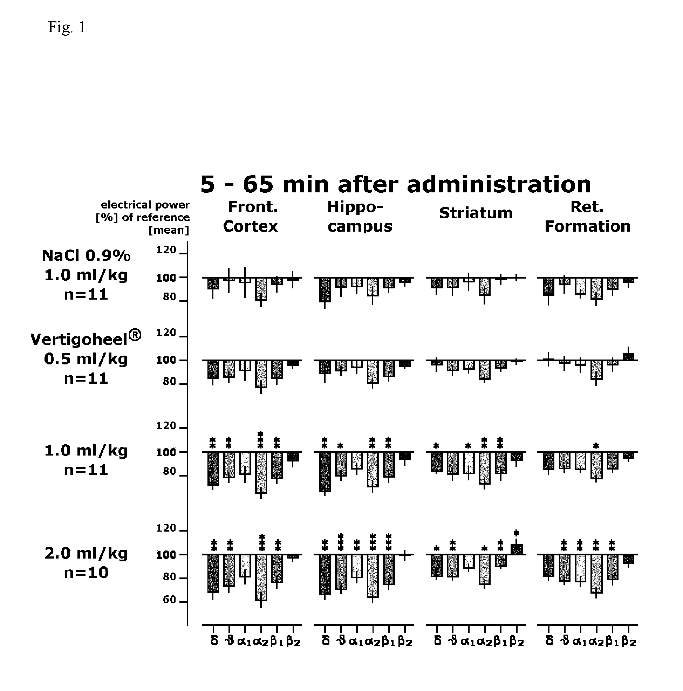

In the presence of 1 mL of Vertigoheel®, alpha2 and delta frequencies were significantly reduced in the frontal cortex, the hippocampus ad the striatum during the first hour of measurement. Within the hippocampus betal waves and within the frontal cortex betal and theta waves were significantly attenuated. During the experiment, the effect of 1 mg of Vertigoheel® faded. The administration of the highest dosage of 2.0 mL/kg led to strong changes of spectral frequency bands, mainly in the hippocampus, frontal cortex and reticular formation. The delta, theta, alpha, and beta waves were significantly decreased. In the striatum a less pronounced effect on the firing rate was observed. The lowest dose of Vertigoheel®, 0.5 mg/kg, did not exhibit any significant changes. Data indicate a dose-and-time-dependent effect of Vertigoheel® on the electrical frequencies (Fig. 1).

Discriminant Analysis of Electropharmacograms: Data from Vertigoheel® were projected in a 3-dimensional coordinate according to their changes of frequency in the EEG. Reference drugs injected intraperitoneally are analyzed during the first half hour after administration. The intraperitoneally administered preparation Vertigoheel® is analyzed for 5 to 65 minutes after administration. Vertigoheel® is represented by 1 dosage of 1 mL/kg. Vertigoheel® positions closely to cognitive-enhancing and analgesic drugs, such as metanicotine and tramadol; and drugs against Parkinson disease, such as rasagiline and selegiline. This indicates a similar drug action like the previously mentioned medications.

This approach provided evidence that Vertigoheel® in ultra higfh dosage affects the CNS directly. It could be shown that Vertigoheel® displays a time and dose dependent effect and mainly affects the frontal cortex and the hippocampus, one of the key brain regions for processing and storage of incoming information (= learning). A further comparison of Vertigoheel® with other pharmacological drugs revealed similarities of our medication to cognitive-enhancing and analgesic drugs, such as metanicotine and tramadol; and drugs against Parkinson disease, such as rasagiline and selegiline. This was the first scientific indication, that Vertigoheel® influences important CNS brain regions involved in cognitive functions.

### Example 2: Hippocampus slice preparation: Effect of Vertigoheel® on neuronal activity in hippocampus

In the first experiment (Tele EEG) Vertigoheel® has been demonstrated to affect the neuronal activity and influence the electrical frequencies in the hippocampus via electroencephalogram measurements. In order to gain further knowledge of a possible direct target of Vertigoheel®, neurophysiologic recordings from hippocampus slices in vitro were performed. The effect of Vertigoheel® on local synaptic circuitry and neuronal activation as well as the effect on different types of glutamate receptors in this cognition relevant area was investigated via this ex vivo system.

Hippocampus slices were obtained from 47 adult male Sprague-Dawlay rats (Charles River Wiga, Sulzbach, Germany). Rats were kept under a reversed day/night cycle for 2 weeks prior to of the experiments, in order to record in vitro activity from slices during the active phase of their circadian rhythm. Animals were exsanguinated under ether anaesthesia, the brain was removed in total and the hippocampal formation was isolated under microstereoscopic sight. Four to five slices were used from each animal. The midsection of the hippocampus was fixed to the table of a vibrating microtome (Rhema Labortechnik, Hofheim, Germany) using a cyanoacrylate adhesive, submerged in chilled bicarbonate-buffered saline (artificial cerebrospinal fluid (ACSF): NaCl: 124 mM, KCl: 5 mM, NaH₂PO4: 1.25 mM; CaCl₂: 2 mM, MgSO₄: 2 mM, NaHCO₃: 26 mM, glucose: 10 mM, and cut into slices of 400 mm thickness. ACSF had an osmolarity of 260-270 mOsmol/l (plasma has an osmolarity of 288+/- 5). All slices were preincubated for at least 1 h in carbogen saturated ACSF (pH 7.4) in a prechamber before use.

During the experiment the slices were held and treated in a special superfusion chamber (List Electronics, Darmstadt, Germany) and kept at 35 °C. Four slices were used from one rat per day under one of the test conditions (control or different concentrations of the test preparation). The preparation was superfused with artificial cerebrospinal fluid (ACSF) at 180-230 ml/h. Electrical stimulation (200 □A constant current pulses of 200 □s pulse width) of the Schaffer Collaterals within the CA2 area and recording of extracellular field potentials from the pyramidal cell layer of CA1 was performed according to conventional electrophysiological methods using the "Labteam" Computer system "NeuroTool" software package (MediSyst GmbH, Linden, Germany). Measurements were performed at 10 min intervals to avoid potentiation mechanisms. Four stimulations - each 20 s apart from each other - were averaged for each time point. After obtaining stable responses to single stimuli (SS) long-term potentiation was induced by applying a theta burst type pattern. The mean amplitude of three signals 20 seconds apart from each other were averaged to give the mean of absolute voltage values (Microvolt) ± standard error of the mean for each experimental condition (single stimulus or theta burst stimulation).

For blocking of glutamate receptors 8 antagonists were used: CGS 19755 (BN0142, 1A/33087), NBQX disodium salt (BN0608, 06088BN/02), GYKI 52466 (BN0380, BN0380, 1A/35052), YM 298198 hydrochloride (BN0553, 0553BN/01), SYM 2206 (0961, 7A/34998), UBP 301 (BN0532, 0532BN/01), (RS)-APICA (BN0092, 0092BN/01), MSOP (BN0349 0349BN/01), all purchased from BIO TREND Chemikalien. They were tested in pilot experiments in order to detect the concentration succeeding in leaving the amplitude of the population spike nearly unchanged in the presence of single stimuli and theta burst stimulation.

### Results

Effect of Vertigoheel® on neuronal activity: Following single electric stimulus application to the Schaffer Collaterals responses of the pyramidal cells increased concentration dependently in the presence of Vertigoheel®. The population spike amplitude increased by about 50% with a maximum at 2 mg/L of Vertigoheel® (amplitude increases from 1,07 to 1,73 mV). Increases were statistically significant different from the control value without drug (p<0.01). Following electric theta burst stimulation the presence of Vertigoheel® only led to insignificant increases of the population spike amplitude (from 2,06 mV to 2,24mV).

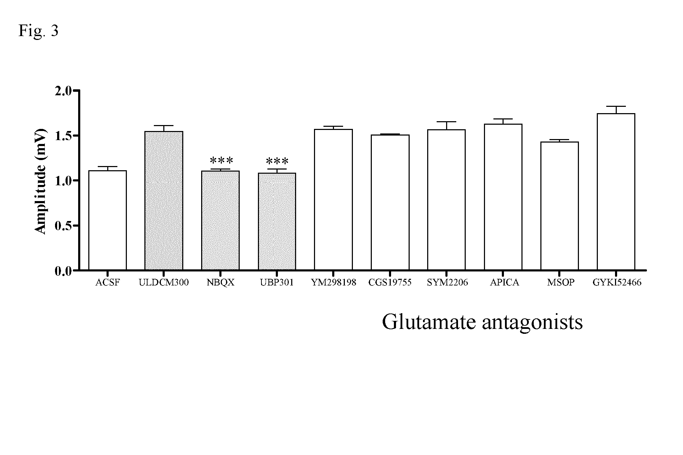

Antagonism of Vertigoheel® by challenge with glutamatergic antagonists: In order to investigate particular targets of Vertigoheel® several glutamate receptor antagonists were added to the superfusion medium of the hippocampus slice. Appropriate dosages were determined via pilot studies. Effective concentrations of Vertigoheel® were taken from the first series of experiments (1ml/L for Vertigoheel®, Fig. 3).

Functional interference with the NMDA receptor mediated signal: In order to test a possible interference of Vertigoheel® with NMDA receptor activated signal changes, the glutamatergic neurotransmission was modulated by CGS 19755, a very potent and selective NMDA receptor antagonist. In the presence of 250 nM CGS 19755 only very minor decreases of the signal amplitude could be measured. A concentration of 250 nM did not increase the amplitude in the presence of Vertigoheel® after single shock stimulation. Thus, no interference with the NMDA receptor mediated signal transmission could be observed after the treatment of Vertigoheel®.

Functional interference with the AMPA receptor mediated signal: In order to test a possible interference of Vertigoheel® with AMPA receptor activated signal changes, the glutamatergic neurotransmission was modulated by NBQX, a very potent and selective competitive AMPA receptor antagonist. In the presence of 50 nM NBQX alone no changes of the signal amplitude could be measured. However, a concentration of 50 nM significantly blocked the enhanced population spike amplitude in the presence of Vertigoheel® after single stimuli. Thus, for Vertigoheel® a clear interference with the AMPA receptor mediated signal transmission could be observed.

Functional interference with non-competitive AMPA receptor modulation by GYKI 52466 and SYM 2206: To test a possible interference of Vertigoheel® with non-competitive AMPA glutamate receptor activated signal changes, the glutamatergic neurotransmission was modulated by GYKI 52466, a very potent and selective non-competitive AMPA receptor antagonist. In the presence of 500 nM GYKI 52466 alone only very minor decreases of the signal amplitude could be measured. GYKI 52466 (500 nM) did not effect the amplitude of Vertigoheel® after single stimuli. Thus, no antagonism with the non-competitive AMPA receptor mediated signal transmission could be observed for Similar effects were obtained in the presence of SYM 2206, another non-competitive AMPA receptor antagonist.

Functional interference with kainate receptor modulation by UBP 301: In order to test a possible interference of Vertigoheel® with kainate receptors, glutamatergic neurotransmission was modulated by UBP 301, a very potent and selective competitive kainate receptor antagonist. In the presence of 50 nM UBP 301 alone no change of the signal amplitude could be measured. The concentration of 50 nM antagonized completely the significant enhancement of the populations spike amplitude in the presence of Vertigoheel® after single stimuli. Thus the data indicate a clear interference of Vertigoheel® with kainate receptors.

Functional interference with metabotropic Glu₁ receptor modulation by YM 298198: To determine a possible interference of Vertigoheel® with the class 1 metabotropic glutamate receptor glutamatergic neurotransmission was modulated by YM 298198, a very potent and selective metabotropic glutamate receptor 1 antagonist. During superfusion with 50 nM YM 298198 alone only a very minor decrease of the signal amplitude after TBS could be measured. Amplitudes following single stimuli were not changed in the presence of Vertigoheel® plus the antagonist. However, during TBS a small increase of the amplitude was seen in the presence of Vertigoheel® plus the antagonist. Thus, Vertigoheel® does not mediate its action via metabotropic glutamate receptor 1.

Functional interference with metabotropic Glu₂ receptor modulation by (RS)-APICA: Class 2 metabotropic glutamate receptor antagonist (RS)-APICA at a concentration of 100 nM alone did not inhibit the signal amplitude of Vertigoheel®. The effect of Vertigoheel® after single shock stimulation was not influenced.

Functional interference with metabotropic Glu₃ receptor modulation by MSOP: Class 3 metabotropic glutamate receptor antagonist MSOP at a concentration of 50 nM alone did not change the signal amplitude after single shock or TBS. The effects of Vertigoheel® after single shock stimulation could not be changed by this compound.

In summary Vertigoheel® showed a very specific target profile. The increased neuronal activity following Vertigoheel® incubation could be antagonized by a competitive AMPA and a kainate receptor antagonist. It is suggested that Vertigoheel® mediates its effect partial via these neuronal receptors, involved in memory and learning processes in the hippocampus.

### Example 3: Effect of Vertigoheel® on neuronal function

The objective of the study was to investigate whether the treatment of different doses of Vertigoheel® have potential effects on neuronal function in vitro. Three different features were investigated: neuronal integrity and viability (MTT and LDH assay), neuronal morphology (neuronal outgrowth of primary hippocampal cells) and influence on APP processing and metabolism (Mesoscale ELISA).

The effect of Vertigoheel® on neuronal integrity and viability was investigated because toxic damage to cells can cause individual cell death and if sufficient numbers of cells are lost, the results can be tissue or organ failure and dysfunction, ultimately leading to death of organism. The effect was investigated in mouse primary hippocampal neurons and human neuroblastoma cell line with LDH and MTT Assays.

Alteration of neuronal and synaptic morphology is essential for neuronal function and might give insights whether Vertigoheel® acts as a cerebral activator and supports neuronal transmission.

The impact on APP processing and other AD relevant metabolism is essential with regard to dementia. Secretases act on the amyloid precursor protein (APP) to cleave the protein into three fragments. Sequential cleavage by β-secretase (BACE) and γ-secretase produces the amyloid-β peptide fragment that aggregates into clumps called "plaques" in the brains of Alzheimer's disease patients. α-secretases act on APP no amyloid-β is formed as α-secretase recognizes a target protein sequence closer to the cell surface than BACE. In the following study the effect of Vertigoheel® on the APP processing and metabolism were investigated.

For primary cell culture C57BL/6J mice at embryonic day 18 are used. Pregnant mice were sacrificed according to the general guidelines for animal experimentation by cervical dislocation. E18 embryos were extracted from the uterus and sacrified by decapitation. The whole brains were removed from the skull and transferred to a culture dish containing ice cold 1x HBSS with phenolred. The next steps were carried out on ice. Hemispheres were separated, the meninges were removed and the hippocampi were dissected from the remaining brain tissue using one fine forceps and a Zeiss Stemi 2000-C stereomicroscope. Isolated hippocampi were collected in a sterile 15 ml tube containing 1x HBSS with phenolred. For dissociation hippocampi were washed with 1x HBSS without phenolred. 1x HBSS was removed to 2 ml and 0.2 ml of 2.5% trypsin was added.

Hippocampi were then incubated and washed. 1x HBSS was removed DNAse I (65 U/ml) was added, hippocampi were mechanically dissociated by pipetting. DMEM adhesion medium supplemented with 10% FCS, 2 mM L-glutamine, 100 IU penicillin and 0.1 mg/ml streptomycin was added. Cell count was determined using an improved Neubauer cell counting chamber. Cell suspension was corrected to the desired density of 10* 104 cells per ml and seeded in 24 well cell culture plates containing poly-1-lysine coated 13 mm round cover slips. The total cell concentration was 2.5 * 104 cells per 1 cm2.

Primary hippocampal neurons were used for the investigation of neuronal integrity and viability and for the investigation of neuronal outgrowth following Vertigoheel® treatment.

The cell line SH-SY5Y is a thrice-cloned neuroblastoma. The cells possess an abnormal chromosome 1, where there is an additional copy of a 1q segment and is referred to trisomy 1q. SH-SY5Y cells are known to be dopamine beta hydroxylase active, acetylcholinergic, glutamatergic and adenosinergic. The cells have very different growth phases. The cells both propagate via mitosis and differentiate by extending neurites to the surrounding area. The dividing cells can form clusters of cells which are reminders of their cancerous nature, but certain treatments such as retinoic acid and BDNF can force the cells to dendrify and differentiate.
The SHSY-5Y were used for investigation of neuronal integrity and viability and investigation of impact on APP processing and metabolism after treatment with Vertigoheel®.

Primary hippocampal neurons were maintained at 37°C and an atmosphere of 5% CO2. Medium was changed 3-18 hrs after plating. DMEM adhesion medium was removed and 600µl of neurobasal culture medium supplemented with 10% B27, 2 mM L-glutamine, 100 U penicillin and 0.1 mg/ml streptomycin was added. Once weekly 200 µl of neurobasal culture medium was added to the wells of a 24 well tissue culture plate and 50µl to the wells of a 96 well tissue culture plate.

SHSY-5Y cells were maintained at 37°C and an atmosphere of 5% CO₂. Medium was changed all 3 days and cells were splitted once a week.

Effect on neuronal integrity and viability measured by the MTT-Assay: The MTT assay is a colorimetric assay for measuring the metabolic activity of enzymes that reduce MTT or close dyes (XTT, MTS, WSTs) to formazan dyes, giving a purple color. A main application allows assessing the viability (cell counting) and the proliferation of cells (cell culture assays). It can also be used to determine cytotoxicity of potential medicinal agents and toxic materials, since those agents would stimulate or inhibit cell viability and growth.

Murine primary hippocampal neurons and SHSY-5Y cells were incubated with Vertigoheel®, Saline Heel and Saline Ulm (see chapter 2.1) for different times in 96 well plates (Table 1a/b) and treated with MTT (3-(4-5-Dimethylthiazol-2y1-2,5-diphenyltetrazoliumbromide) for 3 hours. After addition of acid-isopropanol (0.04 N HCL in isopropanol) to the wells, the plates were read on Power Wave 200, BioTek Instruments using a test wavelength of 570 nm and a reference wavelength of 630 nm. 10 µM rotenone in DMSO is used as positive control and untreated cells are used as negative control.

LDH-Assay: Lactate dehydrogenase (LDH) is a soluble cytosolic enzyme that is released into the culture medium following loss of membrane integrity resulting from either apoptosis or necrosis. LDH activity, therefore, can be used as an indicator of cell membrane integrity and serves as a general means to assess cytotoxicity resulting from chemical compounds or environmental toxic factors.

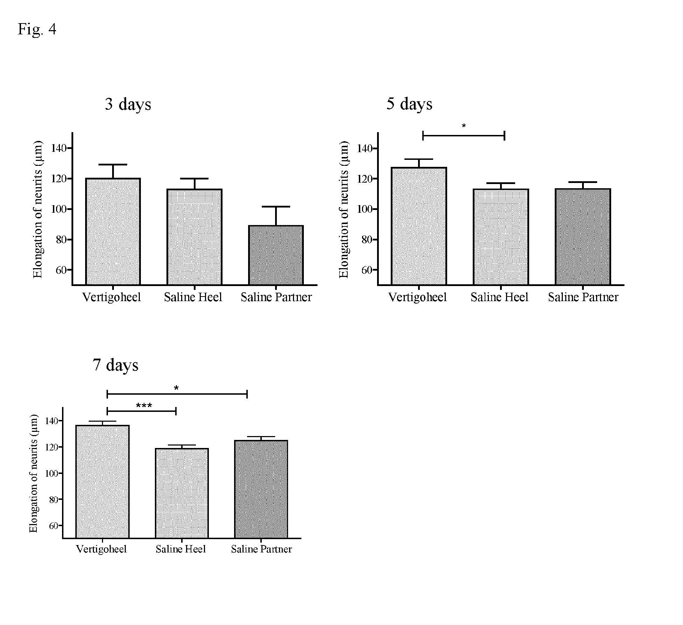

Murine primary hippocampal neurons and SHSY-5Y cells were incubated with Vertigoheel®, Saline Heel and Saline Ulm for different times in 96 well plates (Table 2a/b) and medium supernatant were collected. After addition of tetrazolium salt, plates incubated for 30 minutes. Here the tetrazolium salt is reduced to highly-colored formazan and measured at 490 nm with Power Wave 200, BioTek Instruments (Fig.4). 10 µM rotenone in DMSO is used as positive control and untreated cells are used as negative control.

Alterations of neuronal and synaptic morphology: 8 x 10 ⁴ cells/well murine primary hippocampal neurons were incubated with Vertigoheel®, Saline Heel (Aqua bidest and NaCL purchased by Heel and prepared by University of Ulm) and Saline Ulm (0,9% physiological Saline purchased by Sigma Aldrich) with a concentration of 1:3 and 3:1 for 3 to 7 days to investigate alterations in neurite length and branching to provide insight into proliferative or inhibitory effect of Vertigoheel®. PHN cells were seeded on glas cover slides in 24 well plates, incubated and fixed. Cells were stained with αMAP2 for detection of dendrites and synapses. Using fluorescence microscopy and Sholl analysis changes in length of neurites and number of dendrites are detected.

The impact on APP processing and other AD relevant metabolism was determined next.

Elektrochemoluminescence Assay (sAPP): APP processing: α-secretase and γ-secretase produce non-plaque forming p3, while γ-secretase and β-secretase produce amyloid plaque-forming Aβ. The different regions of the APP protein are indicated.

SHSY-5Y was incubated with Vertigoheel®, Saline Heel and Saline Ulm (Table 3). Medium supernatant is collected and incubated with anti-APP antibody in ELISA format and measured with Sektor Imager 2400 (Mesoscale) to detect changes in APP processing (sAPPs).

To test for significant differences between groups, non-parametric tests were used. For comparison of two groups t-test and non-parametic Wilcoxon rank-sum test were performed (Software: Sigma stat 3.5) for all experiments. Differences with p<0.05 were considered significantly.

### Results

Viability Assay for PHN: MTT assays of PHN demonstrate that cells tolerate a treatment with Vertigoheel® in the dilution of 1:3 and 3:1 up to 5 days. These treatments do not influence the cell viability and proliferation compared to controls The tested dilution of Vertigoheel® and Saline were used for further experiments of neuronal outgrowth measurement. LDH assays produce similar results for usable dilutions of 1:3 and 3:1 in PHNs.

Viability Assay for SHSY-5Y: MTT assays and LDH assays of SHSY-5Y demonstrate that cells well tolerate a treatment with a dilution of 1:5, 1:4, 1:3 and 1:2 and incubation times of 1h, 3h, 5h, 12h and 24h. The cells showed a good viability after treatment. For long time experiments a dilution of 3:1 is tolerable (see report CK3031, University of Ulm).

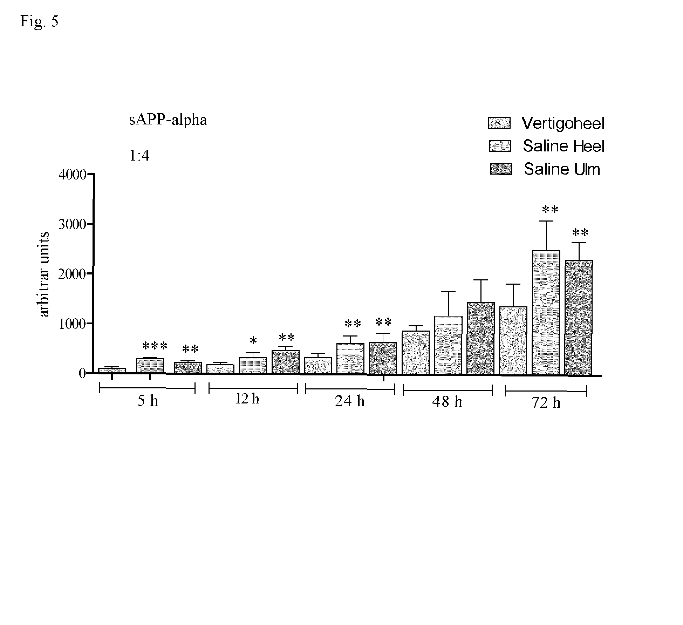

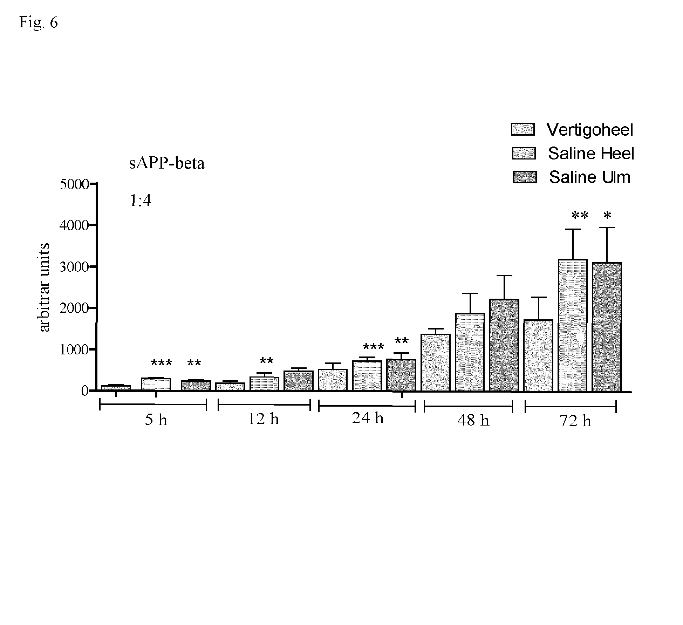

In summary for neuronal outgrowth experiments in PHNs of 3 to 5 days a dilution of 3:1 was used. The investigation of APP processing products in SHSY-5Y will be performed with higher dilution ratios of 1:3 and 1:4. (see also Fig. 4 to 6).

Neuronal and synaptic morphology: Treatment of hippocampal primary neurons with Vertigoheel® over a period of 5-7 days exhibit a significant increase in length of neurites compared to control treated cells. After 3 days of incubation Vertigoheel® treated cells showed an elongation of neurites of 6 µm in average after 5 days 12 µm in average and after 7 days 14 µm in average but only 5 and 7 days are statistically significant.

APP processing: The APP ELISA for SHSY-5Y cells demonstrate a significant decrease of sAPPα and sAPPβ after a incubation time of 5h, 12h, 24h and 72h with a dilution ratio of 1:4 (graph 6 & 7). Long time incubation with a dilution ratio of 3:1 exhibit only a tendency of the decrease of sAPP levels but this effect is increasing with longer incubation time and especially with a daily treatment (data not shown).

sAPP-β and sAPP-α are cleavage products of APP produced by secretases. Physiologically sAPP-α has neurotrophic and neuroprotective properties and increase LTP and spatial memory. Little is know about the role of sAPP- β. However, high levels of amyloid-β (Aβ) protein in the brain have been hypothesized as the primary toxic insult that, via numerous mechanisms, produces cognitive deficits in Alzheimer's disease (AD). It is widely believed that the accumulation of Aβ, a small peptide with a high propensity to form aggregates (plaques), is central to the pathogenesis of AD. Since the amount of both products can be reduced by Vertigoheel®, it is suggested that Vertigoheel® has beneficial effects on the production of this neurotoxic species and therefore might prevents the generation of plaques in the brain.

### Example 4: Effect of Vertigoheel® Scopolamine Induced Contextual Fear Conditioning and Social Transmission of Food Preference Deficit in C57BL/6 Mice

The objective of this study was to investigate whether Vertigoheel® can alleviate scopolamine induced cognitive deficits in male C57BL/6 mice subjected to contextual fear conditioning test (CFC) and social transmission of food preference test (STFP). Both models are commonly used in pharmacological industry to investigate the memory and learning performance in animals after drug treatment. STFP assesses the olfactory memory performance while CFC is determining the effect of Vertigoheel® on the context memory and fear memory.

All animal experiments were carried out according to the National Institute of Health (NIH) guidelines for the care and use of laboratory animals, and approved by the State Provincial Office of Southern Finland, license number 605/01/2006. Altogether 90 male C57BL/6 (Charles River, Germany) at the age of 12 weeks were used for the experiment. Animals were housed at a standard temperature (22 ± 1°C) and in a light-controlled environment (lights on from 7 am to 8 pm) with ad libitum access to food and water.

Animals were grouped as follows:
Phase 1 CFC Test:
   15 mice treated with Vehicle (10 ml/kg, i.p.) for 3 days and Vehicle for scopolamine (10 ml/kg, i.p.) 30 min prior to CFC training
   15 mice treated with Vehicle (10 ml/kg, i.p.) for 3 days and Scopolamine (1 mg/kg, 10 ml/kg, i.p.) 30 min prior to CFC training
   15 mice treated with VERTIGOHEEL® (1 ml/kg, 10 ml/kg, i.p.) and Scopolamine (1 mg/kg, 10 ml/kg, i.p.) 30 min prior to CFC training
   15 mice treated with VERTIGOHEEL® (2 ml/kg, 10 ml/kg, i.p.) and Scopolamine (1 mg/kg, 10 ml/kg, i.p.) 30 min prior to CFC training
   15 mice treated with VERTIGOHEEL® (1 ml/kg, 10 ml/kg, i.p.) for 3 days and Scopolamine (1 mg/kg, 10 ml/kg, i.p.) 30 min prior to CFC training
   15 mice treated with Donepezil (1 mg/kg, 10 ml/kg, i.p.) and Scopolamine (1 mg/kg, 10 ml/kg, i.p.) 30 min prior to CFC training
Phase 2 STFP Test:
   15 mice treated with Vehicle (10 ml/kg, i.p.) for 3 days and Vehicle for scopolamine (10 ml/kg, i.p.) 30 min prior to STFP interaction phase
   15 mice treated with Vehicle (10 ml/kg, i.p.) for 3 days and Scopolamine (1 mg/kg, 10 ml/kg, i.p.) 30 min prior to STFP interaction phase
   15 mice treated with Vertigoheel® (1 ml/kg, 10 ml/kg, i.p.) and Scopolamine (1 mg/kg, 10 ml/kg, i.p.) 30 min prior to STFP interaction phase
   15 mice treated with Vertigoheel® (2 ml/kg, 10 ml/kg, i.p.) and Scopolamine (1 mg/kg, 10 ml/kg, i.p.) 30 min prior to STFP interaction phase
   15 mice treated with Vertigoheel® (1 ml/kg, 10 ml/kg, i.p.) for 3 days and Scopolamine (1 mg/kg, 10 ml/kg, i.p.) 30 min prior to STFP interaction phase
   15 mice treated with Donepezil (1 mg/kg, 10 ml/kg, i.p.) and Scopolamine (1 mg/kg, 10 ml/kg, i.p.) 30 min prior to STFP interaction phase

Scopolamine Administration: Animals were rendered amnesic by an intraperitoneal (i.p.) administration of (-) Scopolamine HBr (1 mg/kg, i.p.). 30 min before CFC test and STFP test.

The ability of the compound Vertigoheel® to reverse the scopolamine-induced amnesia was determined by its single (-30 min) or 3-day administration given prior to CFC training trial. Vehicle (sterile saline) was given i.p. and dosing volume was 10 ml/kg. Donepezil (1 mg/kg) was administered i.p.. To reach the final dosing volume of 10 ml/kg, Vertigoheel® was further diluted with sterile saline 1:4 for the 2 ml/kg group and 1:9 for the 1 ml/kg group. Thus the dose of Vertigoheel® was 1 and 2 ml/kg, but dosing volume 10 ml/kg.

Contextual Fear Conditioning Test: The contextual fear conditioning test (CFC) was modified from Comery et al. (2005). The training and testing were conducted on two consecutive days, using a Coulbourn FreezeFrame system (Coulbourn, Whitehall PA, USA). The CFC test is one of the most commonly used cognitive tests in rodents, namely in the genetically modified mice of Alzheimer's disease. The test is based on the natural behavior of the rodent, i.e. the animal is expected to remain completely immobile (freezing) when facing a fearful situation or stimulus. The involvement of the brain areas responsible for the different aspects of CFC test has been widely examined. It has been discovered that the amygdala is involved in regulating the fear memory (memory for cue and also memory for context) whereas the hippocampus regulates the memory for the context in which the fearful event took place (memory for context) (LeDoux, 1994). Clinically, the CFC test has relevance to human behavior, not only for its cognitive aspects, but also in that fear conditioning can be produced in humans, and damage to the amygdala prevents fear conditioning (LeDoux, 2000). It has also been used as a model for post-traumatic stress disorder (Layton and Krikorian, 2002). Various pharmacological agents, for example estrogen, donepezil and rosiglitazone have been shown to improve the CFC behavior in rodents (Gemma et al., 2004; Dong et al., 2005; Dong et al., 2006).

### Day 1

Training consisted of placing a mouse in a chamber, bright house light on, and allowing exploration for 2 min. Afterward an auditory cue (1700 Hz, 80 dB, conditioned stimulus (CS)) was presented for 15 s. A 2 s foot shock (1.5 mA; unconditioned stimulus (US)) was administered for the final 2 s of the CS. This procedure was repeated, and the mouse was removed from the chamber 30 s later.

### Day 2

About 20 hours after the training, the mouse was returned to the same chamber in which the training occurred (memory for context), and freezing behavior was recorded by a computerized camera tracking system. The automated FreezeFrame system, which digitizes the video signal at 4 Hz and compares movement of the mouse frame by frame, was used to score the amount of freezing. At the end of the 5 min context test, the mouse was returned to its home cage. One hour later, freezing was recorded in a novel environment (altered context) and in response to the cue (memory for cue). The novel environment was the modular test chamber modified by different lighting conditions, colors and textures on the walls and different floor material. The mouse was placed in the novel environment, and time sampling was used to score freezing for 3 min. The auditory cue (1700 Hz, 80 dB, CS) was then presented for 3 min, and freezing was again scored. Freezing scores for each subject are expressed as a percentage for each portion of the test (memory for context, altered context, memory for cue).

Social Transmission of Food Preference in Mice: Social transmission of food preference test was performed 4 weeks after completion of CFC test (wash out period).

The social transmission of food preference (STFP) paradigm involves two socially familiar mice, one designated observer and the other demonstrator. The demonstrator transmits olfactory information about an independent experience with a novel food to the observer during a social interaction subsequent to that experience. The observer is then tested subsequent to the social interaction at a 24h post-interaction for retention of the transmitted odor information: the observer mice are given a choice between the flavor of food eaten by the demonstrator and some other novel flavor. Normal, healthy observer mice will prefer the flavor eaten by the demonstrator. This phenomenon relies on natural behavior of rodents: it is safe to eat a novel food the scent of which has been presented in the breath of a healthy group member. Memory in this task is sensitive to damage of brain regions crucial to memory formation in rodents. Namely, hippocampal lesions prior to training lead to impaired performance (Bunsey and Eichenbaum, 1995) and also the cholinergic basal forebrain has been shown to be critical for successful performance in the task (Berger-Sweeney et al., 2000). The hippocampal memory, crucial for successful performance in the STFP test, could be compared to the declarative memory in humans. This is namely the memory type impaired for example already in the early phase of Alzheimer's disease. For example estrogen and valproic acid have been found to be effective in modulating the STFP performance in rodents (Sgobio et al., 2010; Clipperton et al., 2007).

The protocol was modified from the method description by Wrenn (2004). The mice were group housed, at a ratio of no greater than four observer mice to one demonstrator. Before the experiment, the animals were group fed with unlimited access to ground chow and water. At the end of the group-feeding phase, the ground chow was removed from the cage and replaced by two cups of powdered chow that were placed in the home cages so that the mice were habituated to eat powdered chow from the cups. This habituation phase took 4 days.

### Day 0

After the habituation phase, the demonstrator mouse was removed from the cage and single housed without access to food. The demonstrator mice were not included in any of the treatment groups in the experiment.

### Day 1

The cages of the observer mice were changed to clean ones and all food was removed from the cages. This took place 4 h before the scheduled interaction with the demonstrator mouse.

After a 24 h food deprivation period, each demonstrator mouse was placed in an empty cage with a cup of powdered food, scented either with cinnamon (1 % w/w) or cocoa (2 % w/w). All the mice and groups were divided equally for cinnamon and cocoa -scented food. Each demonstrator mouse was given 1h 30 min to eat the scented food. Immediately after this, they were placed back in their original cages with the observer mice and allowed to interact with their respective observer cage mates mice for 30 min. Demonstrators were then removed and observer mice left undisturbed for 24 h.

### Day 2

24 h after the interaction phase, each observer mouse was then placed in its unique test cage and allowed to eat for 1 h 30 min. Test cages contained one cup of each flavored chow (i.e., cinnamon and cocoa odorized). One of the scents, cocoa or cinnamon, was the "cued food" (presented by the demonstrator 24 h earlier) and the other one was the "non-cued food" (novel scent, not presented earlier). The location of the novel food in the cage (front or rear end of the cage) was counterbalanced across mice. After the 1 h 30 min period, observer mice were returned to their home cages and returned to the free feeding of the ground chow.

For data analysis, all the cups of scented food were weighted before and after the eating period, both for the demonstrator and observer mice. The data are expressed as 1) percent of total intake that was cued food, and 2) as amount (grams) of cued versus non-cued food eaten.

All the compounds (Vehicle, Scopolamine, Donepezil; i.p., 10 ml/kg) were dosed 30 min. prior to interaction phase between the demonstrator and the observer mice on day 1. Vertigoheel® was administered once at the same time as scopolamine, or Vertigoheel® (1 ml/kg, i.p.) was administered for 3 days prior to Day 1 of the STFP test identically as in the CFC test.

All values are presented as mean ± standard deviation (SD) or standard error of mean (SEM), and differences are considered to be statistically significant at the P<0.05 level. Statistical analysis was performed using StatsDirect statistical software. For the CFC test, the differences among means were analyzed by using 1-way-ANOVA followed by Dunnett's post-hoc test (comparison to the control (=vehicle treated scopolamine) group) and by unpaired t-test (vehicle+vehicle vs. scopolamine+vehicle). For the STFP test the amount of cued food vs. non-cued food eaten was analyzed by paired t-test within each treatment group.

### Results

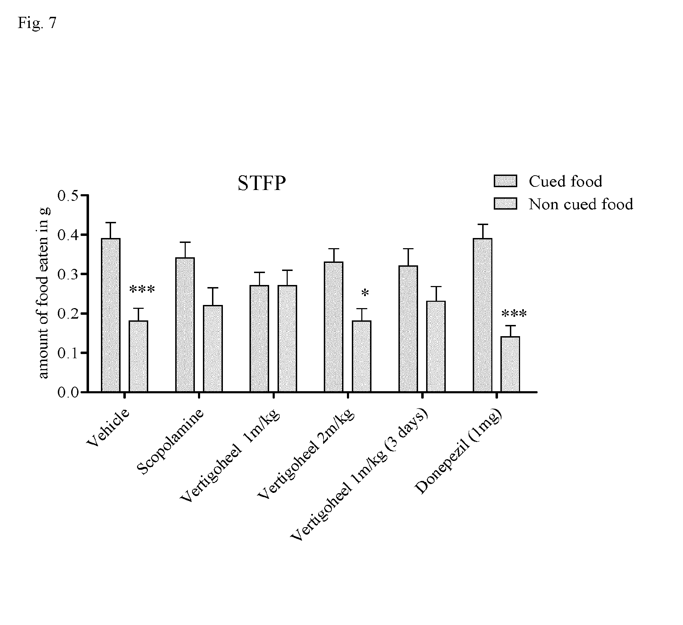

Social transmission of food preference: The contextual fear conditioning test was performed at 12 weeks of age. The effects of Vertigoheel® treatment on freezing behavior in the CFC test are presented in Figure 1. The scopolamine-treated mice spent significantly less time freezing for memory for context compared to vehicle-treated mice, indicating of impaired cognitive functions induced by scopolamine treatment (unpaired t-test, *p<0.05). Within the scopolamine-treated groups there were no significant differences in the freezing scores, although there was a non-significant trend for enhanced freezing in memory for context in the mice treated for 3 days of Vertigoheel® (unpaired t-test: p=0.168) and in the mice treated with donepezil (unpaired t-test: p=0.075), and in memory for cue in the mice treated with a single dose of 1 ml/kg of Vertigoheel® (unpaired t-test: p=0.090) compared to scopolamine-treated mice (One-way ANOVA: p>0.05); see also Fig. 7.

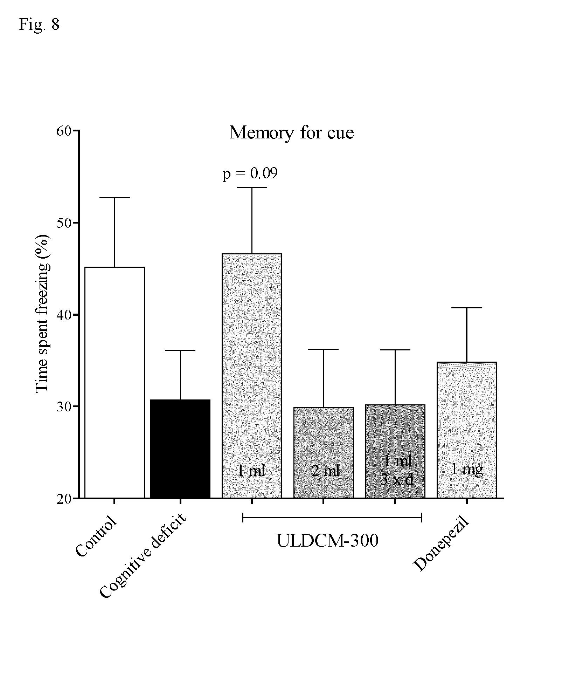

Contextual fear conditioning: The contextual fear conditioning test was performed at 12 weeks of age. The scopolamine-treated mice spent significantly less time freezing for memory for context compared to vehicle-treated mice, indicating of impaired cognitive functions induced by scopolamine treatment (unpaired t-test, *p<0.05, data not shown). Within the scopolamine-treated groups there were no significant differences in the freezing scores, although there was a non-significant trend for enhanced freezing in memory for context in the mice treated for 3 days of Vertigoheel® (unpaired t-test: p=0.168) and in the mice treated with donepezil (unpaired t-test: p=0.075). The effects of Vertigoheel® treatment on freezing behavior in "memory for cue" test are presented in Figure 8. The mice treated with a single dose of 1 ml/kg of Vertigoheel® (unpaired t-test: p=0.090) displayed an increased time spent freezing compared to scopolamine-treated mice (One-way ANOVA: p>0.05), indicating an improved memory performance following Vertigoheel® (see also Fig. 8).

As a summary, the scopolamine treatment caused an impairment in both the context memory in the contextual fear conditioning test and the social transmission of food preference test. In the CFC test there were no significant effects with the Vertigoheel® treatment on the scopolamine-induced memory deficits, although there was a non-significant trend for enhanced context memory in the mice treated for 3 days of 1 ml/kg of Vertigoheel®, and for enhanced cue memory in the mice treated with a single dose of 1 ml/kg of Vertigoheel®. Thus, it is possible, that a single dose treatment with Vertigoheel® influences the amygdaloid functions whereas the longer, 3-day treatment has more potent effects on hippocampal functions. There was a non-significant trend towards enhanced context memory also with the reference compound, donepezil. This partial reversal of the scopolamine-induced amnesia is within the normal range usually observed with donepezil treatment. In the STFP test the scopolamine-induced deficit for the memory of the cued food was reversed in both the mice treated with a single dose of 2 ml/kg of Vertigoheel® and in the mice treated with 1 mg/kg of donepezil. Since approximately 70 % of the cued food eaten of total intake is widely thought to indicate a good preference for the cued food, it is quite common that scopolamine treatment does not result in significant decrease in the percentage amount of cued food eaten of total intake. This is mainly due to the fact that already 50 % is a chance level in the percentage of the cued food eaten. This data provide evidence that Vertigoheel® increases the olfactory memory performance.

### Example 5: The effect of Vertigoheel® on Passive avoidance in scopolamine induced deficits in Wistar rats

The objective of this study was to investigate whether treatment with Vertigoheel® can alleviate cognitive deficits caused by scopolamine administration. Learning and memory deficit was analyzed with passive avoidance (PA) test. This test particularly investigates the long term memory.

All animal experiments were carried out according to the National Institute of Health (NIH) guidelines for the care and use of laboratory animals, and approved by the approved by the State Provincial Office of Southern Finland, license number 605/01/2006 Altogether 90 adult male Wistar rats (Charles River, Germany) weighing 225-350 g were used for the experiment. Animals were housed at a standard temperature (22 ± 1°C) and in a light-controlled environment (lights on from 7 am to 8 pm) with ad libitum access to food and water.

Phase 2 PA Test:
15 rats treated with Vehicle (2 ml/kg, i.p.) for 3 days and Vehicle for scopolamine (2 ml/kg, i.p.) 30 min prior to PA
15 rats treated with Vehicle (2 ml/kg, i.p.) for 3 days and Scopolamine (1 mg/kg, 2 ml/kg, i.p.) 30 min prior to PA
15 rats treated with VERTIGOHEEL® (2 ml/kg, i.p.) once at the testing day together with Scopolamine (1 mg/kg, 2 ml/kg, i.p.) 30 min prior to PA
15 rats treated with VERTIGOHEEL® (1 ml/kg, i.p.) for 3 days and Scopolamine (1 mg/kg, 2 ml/kg, i.p.) 30 min prior to PA
15 rats treated with VERTIGOHEEL® (2 ml/kg, i.p.) for 3 days and Scopolamine (1 mg/kg, 2 ml/kg, i.p.) 30 min prior to PA
15 rats treated with Donepezil (1 mg/kg, 2 ml/kg, i.p.) and Scopolamine (1 mg/kg, 2 ml/kg, i.p.) 30 min prior to PA

Scopolamine Administration: Animals were rendered amnesic by the intraperitoneal (i.p.) administration of scopolamine (1 mg/kg, 2 ml/kg, i.p.) at 30 min prior to T-maze or passive avoidance (PA) training.

Drug Delivery: All the drugs (Vehicle, Scopolamine, Donepezil; i.p., 2 ml/kg) were dosed 30 min prior to PA test. Vertigoheel® (1 or 2 ml/kg) was administered by either given once at the same time as scopolamine, or Vertigoheel® (1 or 2 ml/kg, i.p.) was administered for 3 days prior PA test.

Passive Avoidance: Passive avoidance behavior was tested in Gemini Avoidance system (San Diego Instruments, USA). The behavioral test consisted of a single training trial followed by a test trial 24 h later. For the training trial, each rat was placed in the start chamber for a 15 s adaptation period, after which the start chamber was illuminated, and a guillotine door opened exposing a dark chamber. When the rat entered the dark side, the guillotine door was closed and the animal received a 0.4 mA foot shock for 3 s. Time to enter the dark chamber was recorded. The test trial was performed 24 h later, and was identical to the training trial except that if the rat entered the dark chamber the guillotine door was closed and no shock was delivered. The test had a maximum duration of 600 s on every testing day. Escape latency values from all days as well as the escape latency difference between acquisition and retention trials were used in statistical analyses.

All values were presented as mean ± standard error of mean (SEM), and differences were considered to be statistically significant at the p<0.05 level. Statistical analysis was performed using StatsDirect statistical software. Differences among means were analyzed by using 1-way-ANOVA followed by Dunnet's test (comparison to the control (=vehicle) group) and by un-paired t-test (vehicle+vehicle vs. scopolamine+vehicle). In addition, un-paired t-tests were performed between scopolamine+vehicle and drug+scopolamine groups.

### Results

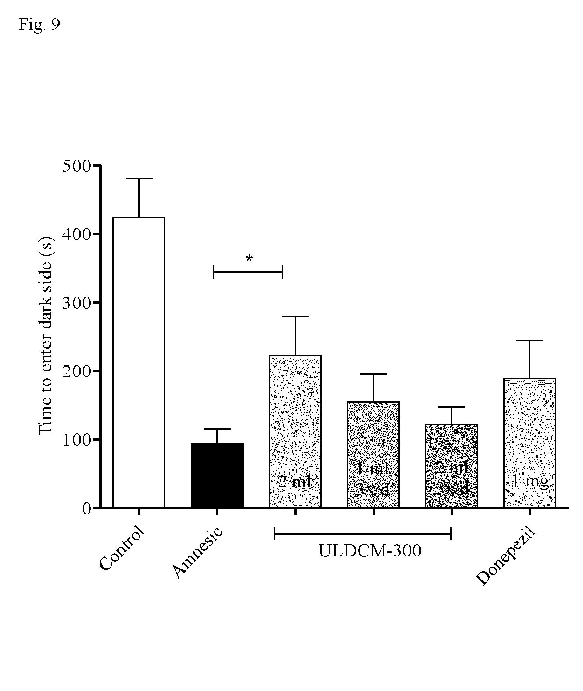

On training day 1, scopolamine treated group did not differ from vehicle treated rats in entering the dark chamber in passive avoidance test when latency time was measured (unpaired t-test, p>0.1). Furthermore, drug treatment groups did not differ from scopolamine group on day 1 indicating the normal behavior of rats. In retention trial on testing day 2, scopolamine treated rats showed a decrease in latency when compared to vehicle treated rats indicating worsened memory retention in the task (unpaired t-test, p<0.0001). However, drug treated groups were ineffective when tested using ANOVA. At a dose of 2 ml/kg, Vertigoheel® could reverse the scopolamine induced memory impairment (unpaired t-test between scopolamine and Vertigoheel® 2 ml/kg group, p<0.05, Fig. 9).

These results suggest that the acute treatment with Vertigoheel® (i.p.) at dose 2.0 ml/kg mildly alleviated memory impairment induced by scopolamine when adult Wistar rats were tested in passive avoidance test. It could be speculated that Vertigoheel® affects hippocampal regions since the function of hippocampus is needed in PA retention.

### Example 6: Effect of Vertigoheel® in the novel object recognition test

The effect of Vertigoheel® was evaluated on cognitive performance of rats assessed using novel object-recognition test. Recognition was measured by the ability of rats to discriminate between a familiar and a new object after a 24 h retention delay to induce natural forgetting in healthy animals. Intraperitoneal administration with Vertigoheel® (0.1, 1 and 2 ml/kg) was performed 40 min before the acquisition trial.

Drug preparation: Vertigoheel® was supplied by Heel as ready made stock solution. All formulations were prepared fresh by Neurofit SAS each day where appropriate. Vertigoheel® was tested at the doses of 0.1, 1 and 2 ml/kg administrated i.p. To obtain the doses of 0.1, 1 and 2 ml/kg in rat using a dosage volume of 2 ml/kg, the stock solution was further diluted 20 and 2 times for 0.1 and 2 ml/kg respectively. Dilution was made in 0.9% NaCl (saline). The treatment was done 40 min prior the acquisition trial. Donepezil was dissolved in saline and given p.o. in a volume of 2 ml/kg. The reference compound was injected 30 min before the acquisition trial.

**Treatment schedule**

| Gr. nr | Description | n | route | Regimen | Time before Acquisition trial (min) |
|---|---|---|---|---|---|
| 1 | Retention after 30min/Saline | 12 | i.p. | acute | 40 |
| 2 | Retention after 24h/Saline | 12 | i.p. | acute | 40 |
| 3 | Retention after 24h /VERTIGOHEEL® (0.1ml/kg) | 12 | i.p. | acute | 40 |
| 4 | Retention after 24h /VERTIGOHEEL® (1ml/kg) | 12 | i.p. | acute | 40 |
| 5 | Retention after 24h /VERTIGOHEEL® (2ml/kg) | 12 | i.p. | acute | 40 |
| 6 | Retention after 24h /Donepezil (0.3 mg/kg) | 12 | i.p. | acute | 30 |

Test animals: 72 male Wistar rats were used for the study. They were purchased from (Janvier; Le Genest St Isle - France). They were group-housed in cages (12 - 14 animals per cage) containing bedding material (Mixal, Safe, Epinay-sur-Orge, France) and maintained in a room with controlled temperature (21-22°C) with relative humidity range of 26 -41 % and a reversed light-dark cycle (12h/12h; lights on: 17:30 - 05:30; lights off: 05:30 - 17:30). Food (A04; Safe, Epinay-sur-Orge, France) and water were available ad libitum.

Apparatus: The apparatus consists of an open acrylic glass cage (100 cm x 100 cm with 45 cm walls) within which animals can move freely. One of the objects is a metallic ball and the other is a black box. The two objects are similar in volume. The animal's approaches to the objects are recorder by a videotracking system (Viewpoint, France).

Experimental procedure: Rats were randomly assigned to one of the different experimental groups. Each animal was identified by its group name, cage number, series (day) of experiment, and number (1 to 10) written with permanent ink on the tail.

The day before testing, the rats were allowed to explore the open-field for 15 min session of habituation. The next day, object A named "familiar object" was placed at the periphery of a virtual central square (30 cm x 30 cm) of the open field. Rats were exposed to this experimental situation (acquisition trial) for 10 minutes. Animals that did not display locomotor activity (total immobility) or do not explore the object are excluded. After 24 h delay to induce natural forgetting (Roncarati et al., 2009), object B ("novel object") was placed adjacent to object A which remained in the same location as during the acquisition trial. Each animal was exposed to this situation for 10 min. The time spent exploring each of the 2 objects during the retention trial (tA and tB, respectively) was recorded separately. Exploration was considered as directing the nose to the objects at a distance ≤ 2 cm to the objects.

Animals that did not display locomotor activity (totally immobile) or did not explore objects more than a total time 5 s (time for object A + time of object B) were excluded.

In one group of rats (group n°1, see Table), the retention trial was performed 30 min post-acquisition instead of 24 h. The performance of these rats was considered as the optimal level of recognition as the spontaneous decay of memory was not present yet.

Calculation: Recognition memory was evaluated using a recognition index (RI) that corresponds to the proportion (%) of time spent on the retention trial investigating object B with respect to total time spent investigating familiar obj ect A and new obj ect B (Bohme et al., 2004). RI was calculated as follow: RI = tB/(tA + tB) x 100, where t denotes time.

Recognition index was also expressed as to the percentage of number of visit (contact) to the novel object corrected for total number of visit to both objects and was calculated as follow: RI = nB/(nA + nB) x 100, where n denotes number of contact.

One-way analysis of variance (ANOVA) followed by Fisher's Protected Least Significant Difference is used to compare pairs of group means. p ≤ 0.05 was deemed significant.

### Results

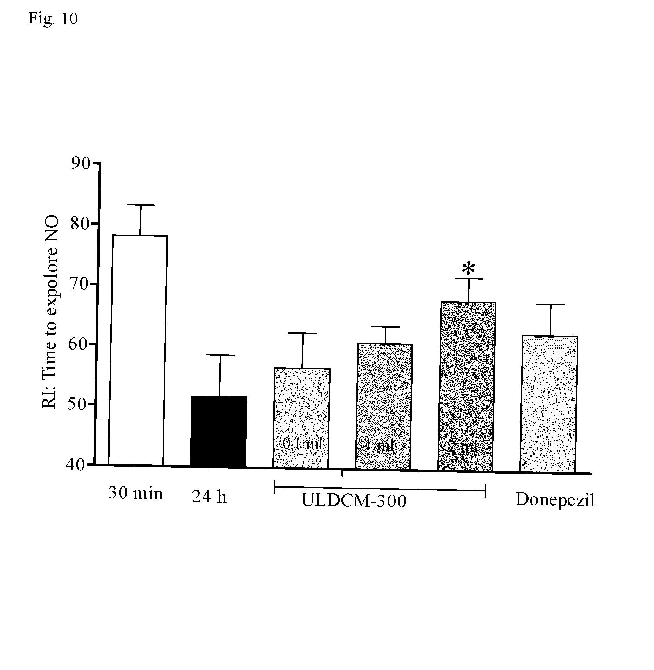

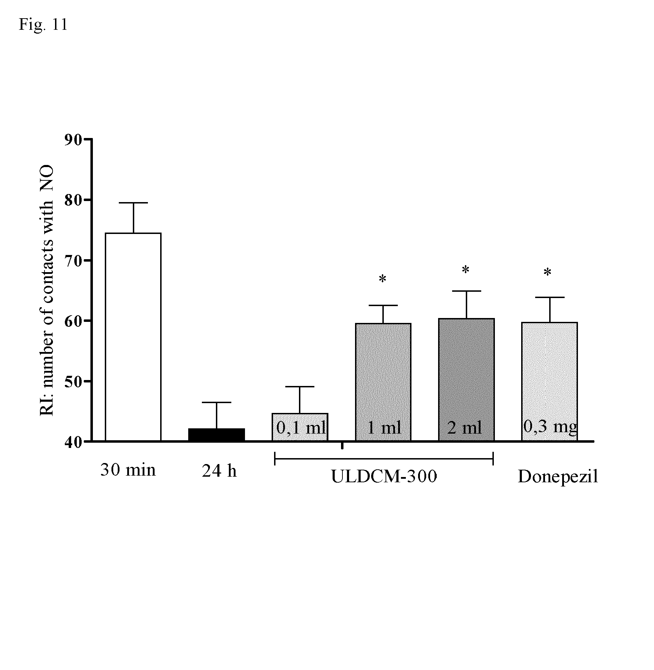

The 24h delay between acquisition and retention trials produced a significant decrease in the recognition of the novel object by the rats. The decrease was 27 and 32% for the time (Fig. 10) and the visit recognition index (Fig. 11), respectively. This natural forgetting was reversed by the treatment with Donepezil. Donepezil was associated with an increase of 11 and 18% in the time and the visit recognition index, respectively. The effect of Donepezil was significant for the visit recognition index.

Vertigoheel® produced a dose-dependent increase in the recognition of the novel object. Indeed, the time recognition index was increased by 5, 9 and 16% for the doses of 0.1, 1 and 2 ml/kg Vertigoheel®, respectively (Fig. 10). The effect was significant for the dose of 2ml/kg (Fig. 11). Furthermore, the visit recognition index was increased by 3, 17 and 18% for the doses of 0.1, 1 and 2 ml/kg Vertigoheel®, respectively. The effect was significant for the doses of 1 and 2 ml/kg.

The present study was undertaken to evaluate the cognitive effect of Vertigoheel® in the novel object recognition test in the rats. Using a protocol of 24 h retention delay to induce natural forgetting, i.p. administration of Vertigoheel® elicited a dose-dependent increase in the recognition of the novel object which suggests a memory enhancing property of the compound. The effect was significant for the doses of 1 and 2 ml/kg.

The object recognition task is a method to measure a specific form of episodic memory in rats and mice. It is based on the spontaneous behavior of rodents and can be considered as a retention test completely free of reference memory components. Moreover, it is not based on usual positive or negative reinforcers such as food or electric shocks, which make the interpretation of drug effects on memory difficult. The object recognition has been predominantly used as a preclinical test to investigate the memory-enhancing effects of acetylcholinesterase inhibitors (AChEIs) such as donepezil, used as treatments for AD. AChE inhibitors had been shown to improve processes of acquisition rather than the consolidation or restitution of object information. In the present study, Vertigoheel® induced a clear improvement in the acquisition of the object information with a comparable degree as of donepezil effect.

### Example 7: Effect of Vertigoheel® in the T-maze alternation task in the mouse

The present work was conducted to evaluate Vertigoheel® for its ability to reverse scopolamine-induced memory deficit in mice as measured by increased alternation in the T-maze test. The T-maze spontaneous alternation is a behavioural test for measuring exploratory behaviour in animals, especially rodent models for CNS disorders. Spontaneous alternation is the innate tendency of rodents to alternate free choices in a T-maze over a series of successive runs. This sequential procedure relies on working memory and is sensitive to various pharmacological manipulations affecting memory processes. This test is based on the willingness of rodents to explore a new environment (e.g. they prefer to visit a new arm of the maze rather than a familiar arm). Many parts of the brain - including the hippocampus, septum, basal forebrain, and prefrontal cortex- are involved in this task. Animals are placed first in the start arm of the maze. The first trial is a forced trial followed by 14 free choice trails, where the animals can chose the left or the right arm. With repeated trials the animals should show less of a tendency to enter a previously visited arm. This test is used to quantify cognitive deficits in mice and evaluate medications for their effects on cognition.

Vertigoheel® was supplied by Heel as ready made stock solution. Vertigoheel® was tested at the doses of 1 and 2 ml/kg administrated i.p. To obtain the doses of 1 and 2 ml/kg in mouse using a dosage volume of 10 ml/kg, the stock solution was further diluted 10 and 5 times, respectively. Dilution was made in 0.9% NaCl (saline).

Donepezil was dissolved in saline and given i.p. in a volume of 10 ml/kg.

All solutions, except the donepezil solution, were prepared and coded by a technician different from the technician who performed the T-maze.

Scopolamine (-(-)scopolamine hydrochloride) was purchased from Sigma (France), dissolved in saline and given i.p. at a dosage volume of 10 ml/kg. Donepezil was a gift from Galantos Pharma GmbH (Germany).

| Group names | Spontaneous | | alternation | |
|---|---|---|---|---|
| | (%) | | | Recovery (%) |
| | Mean | s.e.m. | n | |
| Saline/ vehicle (i.p.) | 66.4 | 2.8 | 10 | 100 |
| Scopolamine 1 mg/kg / vehicle (i.p.) | 32.9 | 1.6 | 10 | 0 |
| Scopolamine 1 mg/kg / Vertigoheel® | | | 10 | |
| (1 ml/kg; i.p., acute) | 57.2 | 2.6 | | 72 |
| Scopolamine 1 mg/kg / Vertigoheel® | | | 10 | |
| (2 ml/kg; i.p., acute) | 57.8 | 2.7 | | 74 |
| Scopolamine 1 mg/kg / Vertigoheel® | | | 10 | |
| (1 ml/kg; i.p., once daily, during 3 days) | 58.5 | 2.3 | | 77 |
| Scopolamine 1 mg/kg / Vertigoheel® | | | 10 | |
| (1 ml/kg; i.p., twice daily, during 3 days) | 57.1 | 3.2 | | 72 |
| Scopolamine 1 mg/kg / Donepezil (0.3 mg/kg;i.p.) | 57.1 | 2.4 | 10 | 72 |

Test animals: Four to five week old male CD-1 mice (Janvier; Le Genest St Isle - France) were used for the study. They were group-housed (10 mice per cage) and maintained in a room with controlled temperature (21-22°C) and a reversed light-dark cycle (12h/12h; lights on: 17:30 - 05:30; lights off: 05:30 - 17:30) with food and water available ad libitum.

Treatment schedule: For acute treatment Vertigoheel® or the vehicle (saline) was i.p. administrated 40 min prior the T-maze trial. For sub-chronic treatment Vertigoheel® or the vehicle (saline) were i.p. administrated during 3 days once or twice a day. The last treatment was done 40 min prior the T-maze trial. Donepezil (0.3 mg/kg) was i.p. administrated 20 min prior the T-maze trial; Scopolamine (1 mg/kg) or its vehicle (saline) was injected i.p. 20 min prior to the T-maze trial.

Experimental procedure: Mice were randomly assigned to one of the different experimental groups. Each animal was identified by its group name, cage number, series (day) of experiment, and number (1 to 10) written with permanent ink on the tail. The T-maze apparatus was made of gray Plexiglas with a main stem (55 cm long x 10 cm wide x 20 cm high) and two arms (30 cm long x 10 cm wide x 20 cm high) positioned at 90 degree angle relative to the main stem. A start box (15 cm long x 10 cm wide) was separated from the main stem by a sliding door. Sliding doors are also provided to close specific arms during the force choice alternation task.

The experimental protocol consists of one single session, which starts with 1 "forced-choice" trial, followed by 14 "free-choice" trials. In the first "forced-choice" trial, the animal is confined 5 s in the start arm and then it is released while either the left or right goal arm is blocked by a horizontal door. After, it will negotiate the maze, eventually enters the open goal arm, and return to the start position. Immediately after the return of the animal to the start position, the left or right goal door is opened and the animal is allowed to choose freely between the left and right goal arm ("free choice trials). The animal is considered as entered in one arm when it places its four paws in the arm. A session is terminated and the animal is removed from the maze as soon as 14 free-choice trials have been performed or 10 min have elapsed, whatever event occurs first.

The apparatus is cleaned between each animal using alcohol (70°). Urine and feces were removed from the maze. During the trials, animal handling and the visibility of the operator are minimized as much as possible.

The percentage of alternation over the 14 free-choice trials was determined for each mouse and was used as an index of working memory performance. This percentage was defined as entry in a different arm of the T-maze over successive trials (i.e., left-right-left-right, etc). Analysis of variance (ANOVA) was performed on the result data. Fisher's Protected Least Significant Difference was used for pairwise comparisons. p value ≤ 0.05 were considered significant. The drug induced reversion of scopolamine-induced memory deficit was calculated by setting the respective response of the saline/vehicle as 100% and that of the group scopolamine/vehicle as 0% reversion.

### Results

A concentration of 1, 1 mg/kg scopolamine was associated with significant reduction of spontaneous alternation (about 34% reduction) as compared to the performance of saline-injected mice.

The scopolamine-induced alternation disruption was reversed by the treatment with 0.3 mg/kg donepezil with an increase of alternation of 24%. The degree of recovery in the performance of mice associated with donepezil treatment was 72%.

Treatment with Vertigoheel® elicited an increase in the alternation of scopolamine-treated mice. The increase was 24 and 25% for the acute doses of 1 and 2 ml/kg, respectively. The recovery in the cognitive performance was 72 and 74% for the doses of 1 and 2 ml/kg, respectively. The increase was 26 and 24% the sub-chronic treatment at 1 ml/kg once or twice a day, respectively. The recovery in the cognitive performance was 77 and 72% the sub-chronic treatment at 1 ml/kg once or twice a day, respectively. There was no statistical difference between the effects induced by the different treatment with Vertigoheel®.

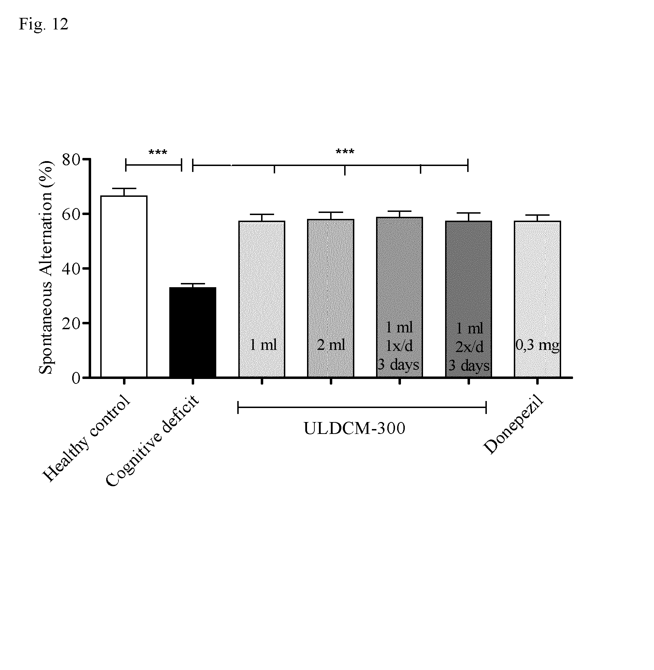

The change induced by Vertigoheel® was not statistically different with that of scopolamine / donepezil group (see also Fig. 12).

In the present study, we sought to evaluate the cognitive enhancing property of Vertigoheel®. Investigations were conducted in scopolamine-treated mice by measuring the increase in the spontaneous alternation in T-maze.

In line with literature findings, donepezil produced a significant increase in the spontaneous alternation of scopolamine -treated mice which suggests an improved memory performance in the T-maze assay.

Vertigoheel® elicited a significant increase in the spontaneous alternation of scopolamine - treated mice, suggesting that Vertigoheel® improves the memory performance of scopolamine-treated mice in the T-maze assay. Furthermore, the effect of Vertigoheel® was comparable with that of 0.3 mg/kg donepezil.

It was observed that single 1h pre-treatment with Vertigoheel® is sufficient to produce an effect comparable with that of sub-chronic treatment. It is thus very plausible that Vertigoheel® contains fast-acting principle(s) responsible for the improvement of the cognitive function of mice in the T-maze paradigm. Furthermore, it appears that the effect was already optimal after a single treatment with Vertigoheel®. However, it is not known yet whether greater efficacy could be obtain with lower dose range. Additional experiments are needed to address this latter point.

These findings support the role of ultra high dosages of Vertigoheel® as a cerebral active preparation, associated with enhanced neuronal processing efficiency and memory performance. The data provide mechanistic evidence for the enhanced cognitive activity of Vertigoheel®. Thus, Vertigoheel® is a potential preparation which can be used as an adjunctive pharmacologic therapy in dementia.

## Claims

1. A composition for use in treating and/or preventing a neurodegenerative disorder or disease, wherein said composition comprises extracts of Conium maculatum obtainable according to method 2a of the German Homeopathic Pharmacopeia, Ambra grisea obtainable according to the information in the Ambra grisea monograph of the German Homeopathic Pharmacopeia, Petroleum rectificatum obtainable according to method 5a and additional information in the Petroleum rectificatum monograph of the German Homeopathic Pharmacopeia, and Anamirta cocculus obtainable according to method 4a and additional information in the Anamirta cocculus monograph of the German Homeopathic Pharmacopeia and wherein the composition provides the said extracts in a dosage of at least about 1 ml/kg body weight of the subject to be treated per day.

2. The composition of claim 1, wherein said composition is comprising the said raw extracts is to be administered in a concentration of at least about 1 ml/kg to at least about 2ml/kg body weight of the subject to be treated per day.

3. The composition of claim 1 or 2, wherein said neurodegenerative disorder or disease is associated with cognitive impairment.

4. The composition of claim 3, wherein said cognitive impairment is prevented or ameliorated.

5. The composition of any one of claims 1 to 4, wherein said neurodegenerative disease or disorder is Alzheimer's disease.

6. The composition of claim 5, wherein amyloid-beta precursor protein (APP) processing accompanying Alzheimer's disease is reduced.

7. The composition of any one of claims 1 to 43, wherein said neurodegenerative disease or disorder is amnesia, learning and memory deficits, amyotrophic lateral sclerosis, circumscribed brain atrophy, corticobasale degeneration, degeneration of nervous system due to alcohol, unspecified degenerative disease of nervous system, degenerative syndrome, diabetic polyneuropathy, epilepsy, Friedreich's ataxias, frontotoemporale dementia and Parkinsonism of Chromosom 17, Huntington's disease, mild cognitive impairment, Morbus Pick, non-classified degenerative diseases or disorders of nervous system, progressive isolated aphasia, progressive supranuclear palsy, non-classified senile degeneration of brain, spinocerebellar ataxias/degenerations, mental retardation, spinal cord injury, forgetfullness, and Parkinson's disease.

8. The composition of any one of claims 1 to 7, wherein neuronal outgrowth is increased upon administration of the composition.

9. The composition of any one of claims 1 to 8, wherein said composition comprises at least one further pharmaceutically active ingredient selected from the group consisting of:
Donepezil, Rivastigmine, Galantamine, Tacrine , Mementine, Captopril, Etanercept, Gingko Biloba, Vitamine B12, Resveratrol, Ginseng, Green Tea Extract, Vitamine E, Levodopa, Pramipexole dihydrochloride monohydrate, and Rasagiline.

10. A composition for use in improving learning and memory, wherein said composition is a composition which comprises extracts of Conium maculatum obtainable according to method 2a of the German Homeopathic Pharmacopeia, Ambra grisea obtainable according to the information in the Ambra grisea monograph of the German Homeopathic Pharmacopeia, Petroleum rectificatum obtainable according to method 5a and additional information in the Petroleum rectificatum monograph of the German Homeopathic Pharmacopeia, and Anamirta cocculus obtainable according to method 4a and additional information in the Anamirta cocculus monograph of the German Homeopathic Pharmacopeia and wherein the composition provides the said extracts in a dosage of at least about 1 ml/kg body weight of the subject to be treated per day.

11. The composition of claim 10, wherein natural forgetting is ameliorated in order to improve learning and memory.

12. The composition of any one of claims 1 to 11, wherein said composition further comprises a pharmaceutically acceptable carrier and/or diluent.

13. A medicament comprising a composition which comprises extracts of Conium maculatum obtainable according to method 2a of the German Homeopathic Pharmacopeia, Ambra grisea obtainable according to the information in the Ambra grisea monograph of the German Homeopathic Pharmacopeia, Petroleum rectificatum obtainable according to method 5a and additional information in the Petroleum rectificatum monograph of the German Homeopathic Pharmacopeia, and Anamirta cocculus obtainable according to method 4a and additional information in the Anamirta cocculus monograph of the German Homeopathic Pharmacopeia and wherein the composition provides the said extracts in a dosage of at least about 1 ml/kg body weight of the subject to be treated per day.

14. The medicament of claim 13, wherein said medicament further comprises a pharmaceutically acceptable carrier and/or diluent.

15. A method for the manufacture of a medicament, preferably, for use in treating and/or preventing a neurodegenerative disorder or disease comprising the steps of:
(a) preparing a composition which comprises extracts of Conium maculatum obtainable according to method 2a of the German Homeopathic Pharmacopeia, Ambra grisea obtainable according to the information in the Ambra grisea monograph of the German Homeopathic Pharmacopeia, Petroleum rectificatum obtainable according to method 5a and additional information in the Petroleum rectificatum monograph of the German Homeopathic Pharmacopeia, and Anamirta cocculus obtainable according to method 4a and additional information in the Anamirta cocculus monograph of the German Homeopathic Pharmacopeia and wherein the composition provides the said extracts in a dosage of at least about 1 ml/kg body weight of the subject to be treated per day; and
(b) formulating said composition as a medicament in a pharmaceutically acceptable manner.
